# EUROPÄISCHE PATENTSCHRIFT

(11) **EP 4 201 459 B1**
(45) Veröffentlichungstag und Bekanntmachung des Hinweises auf die Patenterteilung: **12.03.2025**
(21) Anmeldenummer: 22212815.9
(22) Anmeldetag: 12.12.2022
(51) Int. Cl.: A61M 16/01, A61M 16/18

(54) **NARKOSEMITTELDOSIERER MIT EINEM BESCHICHTETEN NARKOSEMITTELBEHÄLTER UND HERSTELLUNGS-VERFAHREN**
ANESTHETIC DOSING DEVICE WITH A COATED ANESTHETIC CONTAINER AND METHOD OF MANUFACTURE
DISPOSITIF DE DOSAGE D'ANESTHÉSIQUE AVEC UN RÉCIPIENT D'ANESTHÉSIQUE REVÊTU ET PROCÉDÉ DE FABRICATION

(30) Priorität: 21.12.2021 DE 102021134138
(43) Veröffentlichungstag der Anmeldung: 28.06.2023
(73) Patentinhaber: Drägerwerk AG & Co. KGaA, 23558 Lübeck (DE)
(72) Erfinder: RADOMSKI, Klaus, 23558 Lübeck (DE); PASDZIOR, Sven, 23558 Lübeck (DE); REINSCHMIEDTH, Sascha, 23558 Lübeck (DE)

(56) Entgegenhaltungen:
- EP-A1- 0 366 268
- WO-A1-00/73722
- WO-A1-2010/129796
- WO-A1-2020/146919
- US-A- 4 072 243

## Beschreibung

Die Erfindung betrifft einen Narkosemitteldosierer mit einem beschichteten Narkosemittelbehälter und ein System zur künstlichen Beatmung und Narkotisierung eines Patienten mit einem solchen Narkosemitteldosierer. Weiterhin betrifft die Erfindung ein Herstellungs-Verfahren zum Herstellen eines derartigen Narkosemitteldosierers.

Um einen Patienten zu sedieren oder zu narkotisieren, wird dem Patienten in der Regel ein Gasgemisch zugeführt, welches Sauerstoff und mindestens ein Narkosemittel umfasst. Am oder im Körper des Patienten ist wenigstens zeitweise eine patientenseitige Koppeleinheit angebracht, beispielsweise eine Atemmaske oder ein Tubus oder ein Katheter. In vielen Fällen führt ein Anästhesiegerät eine Abfolge von Beatmungshüben durch. In jedem Beatmungshub wird jeweils eine Menge dieses Gasgemischs zu der patientenseitigen Koppeleinheit gefördert.

In vielen Fällen steht eine Förderereinheit des Anästhesiegeräts in einer Fluidverbindung mit einem Narkosemittelverdampfer. Dieser Narkosemittelverdampfer erhält flüssiges Narkosemittel aus einem Narkosemittelbehälter, verdampft oder verdunstet dieses und liefert dadurch gasförmiges Narkosemittel. Häufig gehört der Narkosemittelverdampfer zu einem Gasgemisch-Erzeuger, und das gasförmige Narkosemittel wird in einer Mischkammer des Gasgemisch-Erzeugers mit einem Trägergas vermischt und in den Gasfluss eingespeist, den die Förderereinheit zur patientenseitigen Koppeleinheit fördert.

Das flüssige Narkosemittel wird in einem Narkosemittelbehälter mit einem Narkosemitteltank vorrätig gehalten. Von Zeit zu Zeit ist es erforderlich, flüssiges Narkosemittel in den Narkosemitteltank nachzufüllen. Ein solcher Narkosemittelbehälter wird beispielsweise in DE 10 2004 041 448 B3 beschrieben. Die Wandung des Narkosemitteltanks ist aus einem Werkstoff, der Aluminium enthält, hergestellt.

In WO 00 / 73 722 A1 wird ein System (gas delivery system 10) beschrieben, das ätzende (corrosive) Gase für die Herstellung von Halbleiterbauteilen bereitzustellen vermag. Diese Gase können hydrolysieren und mit metallischen Oberflächen reagieren. Die nach innen zeigende Oberfläche eines Gaszylinders 11 ist mit einer Beschichtung aus Nickel und Phosphor versehen, wobei die Dicke der Beschichtung höchstens 20 µm beträgt. In einer Ausführungsform liegt der Anteil von Phosphor zwischen 1 Gew-% und 15 Gew-%. Der Nickelanteil liegt zwischen 80 Gew-% und 97 Gew-%. Diese Beschichtung kann durch einen elektrolosen Prozess hergestellt werden.

WO 2020 / 146 919 A1 zeigt einen Narkosemitteldosierer (anesthetic vaporizer 1) mit einem internen Reservoir, einem externen Behälter (bucket 3) und einer Nachfülleinheit (filler 2). Die Wand des internen Reservoirs und die Wand des externen Behälters 3 sind aus Aluminium hergestellt. Auf die innere Oberfläche ist eine Beschichtung aus Nickel aufgetragen, um die Gefahr zu reduzieren, dass das Aluminium korrodiert. Diese Beschichtung ist für übliche Narkosemittel undurchlässig. In die Wand der Nachfülleinheit 2 ist ein Sichtglas (fluid level sightglass 20) für den Füllstand eingelassen.

In WO 2010 / 129 796 A1 wird ein Narkosemittelbehälter (anesthetic container 50) für einen Narkosemitteldosierer (vaporizer) beschrieben. Der Narkosemittelbehälter 50 umfasst einen Narkosemitteltank (receptacle 52) in Form einer Flasche. Auf die innere Oberfläche der Wand 54 ist eine Beschichtung 70 aus einer Legierung (alloy) aufgetragen. Diese Legierung kann Nickel oder eine Nickelverbindung umfassen.

Der Erfindung liegt die Aufgabe zugrunde, einen Narkosemitteldosierer mit den Merkmalen des Oberbegriffs des Anspruchs 1 bereitzustellen, wobei der Narkosemittelbehälter eine höhere Zuverlässigkeit als bekannte Narkosemitteldosierer bieten soll. Außerdem liegt der Erfindung die Aufgabe zugrunde, ein Herstellungs-Verfahren zur Herstellung eines solchen Narkosemitteldosierers bereitzustellen, welches zuverlässiger als bekannte Herstellungs-Verfahren ist.

Die Aufgabe wird durch einen Narkosemitteldosierer mit den Merkmalen des Anspruchs 1 und durch ein Herstellungs-Verfahren mit den Merkmalen des Anspruchs 10 gelöst. Merkmale und vorteilhafte Ausgestaltungen des Herstellungs-Verfahrens sind, soweit sinnvoll, auch vorteilhafte Ausgestaltungen des erfindungsgemäßen Narkosemitteldosierers und umgekehrt.

Der erfindungsgemäße Narkosemitteldosierer umfasst einen Narkosemittelbehälter. Unter einem Narkosemitteldosierer wird ein Bauteil mit einem Narkosemittelbehälter verstanden, welches im Narkosemittelbehälter ein flüssiges Narkosemittel speichert und unter Verwendung des gespeicherten flüssigen Narkosemittels gasförmiges Narkosemittel erzeugt und bereitstellt. Der erfindungsgemäße Narkosemitteldosierer umfasst einen solchen Narkosemittelbehälter . Der Narkosemitteldosierer kann mindestens zwei Narkosemittelbehälter umfassen, insbesondere für unterschiedliche Narkosemittel, wobei mindestens ein Behälter erfindungsgemäß ausgestaltet ist, bevorzugt beide. Der Narkosemitteldosierer umfasst einen Narkosemittelverdampfer, und der Narkosemittelverdampfer vermag ein gasförmiges Narkosemittel zu erzeugen und hierbei flüssiges Narkosemittel aus dem Narkosemittelbehälter zu verwenden.

Der erfindungsgemäße Narkosemittelbehälter umfasst einen Narkosemitteltank. Dieser Narkosemitteltank umschließt einen Innenraum und vermag in diesem Innenraum ein flüssiges Narkosemittel aufzunehmen. Weiterhin umfasst der Narkosemittelbehälter eine Nachfülleinheit, insbesondere einen Stutzen oder eine Nachfüllöffnung. Durch diese Nachfülleinheit hindurch lässt sich flüssiges Narkosemittel in den Narkosemitteltank nachfüllen. Bevorzugt lässt die Nachfülleinheit sich mit einem Verschluss fluiddicht verschließen. Um flüssiges Narkosemittel nachzufüllen, lässt der Verschluss sich von der Nachfülleinheit entfernen und später wieder aufsetzen.

Anmerkung: Möglich ist, dass ein Teil des flüssigen Narkosemittels bereits beim Nachfüllen oder später im Narkosemitteltank verdampft. Der übrige Teil des Narkosemittels gelangt beim Nachfüllen in einem flüssigen Zustand in die Narkosemitteltank. Wenigstens ein Teil dieses Narkosemittels im Narkosemitteltank bleibt aber auch nach dem Nachfüllen flüssig.

Der Narkosemitteltank umfasst eine Wandung. Auf die innere Oberfläche der Wandung des Narkosemitteltanks ist eine Beschichtung aufgebracht. Diese innere Oberfläche zeigt zum Innenraum und somit zum flüssigen Narkosemittel im Narkosemitteltank hin. Die Beschichtung befindet sich also zwischen der Wandung und dem Innenraum mit dem flüssigen Narkosemittel. Bevorzugt ist die Beschichtung so ausgestaltet, dass die Wandung an keiner Stelle direkt mit dem flüssigen Narkosemittel in Kontakt kommt, sondern idealerweise an jeder Stelle die Beschichtung dazwischen ist.

Die Beschichtung auf der inneren Oberfläche des Narkosemitteltanks ist aus einer Legierung hergestellt. Diese Legierung umfasst Nickel (Ni) und Phosphor (P). Der Anteil von Nickel in der Legierung liegt zwischen 80 Gew-% und 97 Gew-%, bevorzugt zwischen 85 Gew-% und 90 Gew-%. Der Anteil von Phosphor in der Legierung liegt zwischen 3 Gew-% und 15 Gew-%, bevorzugt zwischen 10 Gew-% und 13 Gew-%. Selbstverständlich beträgt die Summe des Nickel-Anteils und des Phosphor-Anteils höchstens 100 Gew-%.

Die erfindungsgemäße Beschichtung auf der inneren Oberfläche des Narkosemitteltanks reduziert das Risiko, dass der Werkstoff der Wandung chemisch auf ein Narkosemittel im Narkosemitteltank einwirkt. Diese chemische Einwirkung könnte das Narkosemittel in unerwünschter Weise verändern. Die Beschichtung verhindert oder reduziert wenigstens das Risiko, dass die Wandung in Kontakt mit dem Narkosemittel kommt und dadurch eine unerwünschte chemische Einwirkung auf das Narkosemittel stattfindet.

Erfindungsgemäß umfasst die Beschichtung zwischen der Wandung und dem Innenraum eine Legierung aus Nickel und Phosphor. Diese Legierung reagiert nur in relativ geringem Maße chemisch mit einem flüssigen oder auch gasförmigen Narkosemittel. Daher vermag der Narkosemitteltank flüssiges Narkosemittel vorrätig zu halten, auch über einen längeren Zeitraum hinweg und auch bei höheren Umgebungstemperaturen, ohne dass sich das Narkosemittel wesentlich chemisch ändert. Diese Eigenschaft ist insbesondere dann wichtig, wenn ein Narkosemitteldosierer mit einem erfindungsgemäßen Narkosemittelbehälter in Reserve gehalten wird und erst dann verwendet wird, wenn ein anderer Narkosemitteldosierer kein gasförmiges Narkosemittel mehr bereitzustellen vermag. Insbesondere während einer laufenden Narkotisierung eines Patienten ist es nämlich in vielen Fällen erforderlich, von einem Narkosemitteldosierer mit einem leeren Narkosemitteltank auf einen anderen Narkosemitteldosierer umzuschalten, der zuvor in Reserve gehalten wurde, möglicherweise über einen längeren Zeitraum.

Darüber hinaus reduziert die Beschichtung auf der inneren Oberfläche das Risiko, dass ein flüssiges Narkosemittel im Narkosemitteltank die Wandung chemisch angreift. Das könnte zu einem Leck In der Wandung führen, was wiederum oft die unerwünschte Folge hat, das Narkosemittel in die Umgebung austritt.

Die Legierung aus Nickel und Phosphor weist in der Regel eine ausreichende mechanische Beständigkeit auf. In vielen Fällen bleibt daher die Beschichtung auch dann erhalten, wenn der Narkosemittelbehälter mechanischen Einwirkungen von außen, beispielsweise Stößen oder Erschütterungen, und / oder wechselnden Umgebungstemperaturen ausgesetzt ist.

Die Wandung des Narkosemitteltanks kann eine relativ komplizierte Geometrie aufweisen und insbesondere Ecken und / oder Kanten mit einem geringen Krümmungsradius und / oder Hinterschneidungen und / oder Einbuchtungen umfassen. In vielen Fällen lässt sich dank der Erfindung auch bei einer relativ komplizierten Geometrie eine gleichmäßige Beschichtung der inneren Oberfläche des Narkosemitteltanks erzielen. "Gleichmäßig" bedeutet insbesondere, dass alle Bereiche der inneren Oberfläche mit der Beschichtung bedeckt sind und idealerweise gar keine Kontaktfläche auftritt, in der ein Narkosemittel im Narkosemitteltank einen direkten Kontakt zur Wandung hat. In der Praxis lässt sich in vielen Fällen erreichen, dass die nach der Beschichtung verbleibende Kontaktfläche höchstens 5 %, in vielen Fällen sogar höchstens 1 %, der gesamten Fläche der inneren Oberfläche aufweist.

In vielen Fällen ist es möglich, die Beschichtung so auf die Wandung aufzubringen, dass die Beschichtung eine gewünschte und eine relativ gleichmäßige Schichtdicke aufweist, d.h. die Schichtdicke variiert um nicht mehr als ± 5 µm um einen gewünschten mittleren Schichtdicken-Wert. Diese Beschichtung mit einer gleichmäßigen Schichtdicke lässt sich in vielen Fällen auch dann erzielen, wenn die Wandung des Narkosemitteltanks die gerade erwähnte relativ komplizierte Geometrie aufweist. Außerdem führt eine erfindungsgemäße Beschichtung häufig zu einer besonders glatten Oberfläche. Eine glatte Oberfläche reduziert die effektive Fläche und reduziert weiter das Risiko, dass das Narkosemittel im Narkosemitteltank in einen direkten Kontakt mit der Wandung kommt.

Bevorzugt weist die Beschichtung eine Schichtdicke zwischen 0,5 µm und 80 µm auf, besonders bevorzugt zwischen 10 µm und 20 µm.

Erfindungsgemäß liegt der Anteil von Phosphor (P) in der Legierung zwischen 3 Gew-% und 15 Gew-%. Bevorzugt liegt dieser Anteil zwischen 10 Gew-% und 13 Gew-%. Bei einem Phosphor-Anteil oberhalb von 10 Gew-% weist die Beschichtung ein amorphes Gefüge aus. Dieses Merkmal reduziert die Gefahr, dass in der Beschichtung Inhomogenitäten auftreten, beispielsweise Korngrenzen oder ausgeschiedene Phasen. Solche Inhomogenitäten können die mechanische Beständigkeit der Beschichtung reduzieren. Weiterhin reduziert ein Phosphor-Anteil oberhalb von 10 Gew-% aufgrund des erzielten amorphen Gefüges das Risiko, dass in der Beschichtung Kristalle und / oder große Poren auftreten. Sowohl Kristalle als auch Poren können zu Lücken in der Beschichtung führen und dadurch bewirken, dass eine größere Menge des Narkosemittels in direktem Kontakt mit der Wandung kommt. Wie oben bereits dargelegt, ist dies unerwünscht.

In der Regel fließt beim Nachfüllen flüssiges Narkosemittel durch die Nachfülleinheit in den Narkosemitteltank. In einer Ausgestaltung umfasst die Nachfülleinheit ebenfalls eine Wandung, insbesondere dann, wenn die Nachfülleinheit als Stutzen ausgestaltet ist. Die Wandung der Nachfülleinheit ist fluiddicht mit der Wandung des Narkosemitteltanks verbunden. Möglich ist, dass die Wandung des Narkosemitteltanks und die Wandung der Nachfülleinheit ein einziges Bauteil bilden. Bevorzugt sind die beiden Wandungen aus einem starren Werkstoff hergestellt.

Möglich ist, dass auf die innere Oberfläche der Wandung der Nachfülleinheit ebenfalls eine Beschichtung aufgebracht ist. Möglich ist auch, dass auf die äußere Oberfläche der Wandung des Narkosemitteltanks und / oder der Nachfülleinheit ebenfalls eine Beschichtung aufgebracht ist.

In einer Ausgestaltung ist die innere Oberfläche der Wandung der Nachfülleinheit nicht beschichtet. Eine solche Beschichtung ist in manchen Fällen nicht erforderlich, insbesondere dann, wenn der Spiegel des flüssigen Narkosemittels im Narkosemitteltank unterhalb der Nachfülleinheit bleibt und daher nur beim Nachfüllen die Nachfülleinheit in Kontakt mit Narkosemittel kommt. In einer anderen Ausgestaltung sind die innere Oberfläche der Wandung der Nachfülleinheit und die innere Oberfläche der Wandung des Narkosemitteltanks mit einer Beschichtung aus dem gleichen Werkstoff versehen. Besonders bevorzugt ist die gesamte innere Oberfläche der Wandung des Narkosemittelbehälters von einer durchgehenden Beschichtung überdeckt - bis auf die innere Oberfläche eines optionalen Verschlusses für die Nachfülleinheit und bis auf eine nachfolgend beschriebene optionale visuelle Inspektionseinheit. Dadurch wird ein unerwünschter Kontakt zwischen der Wandung und dem Narkosemittel auch dann verhindert, wenn der Narkosemittelbehälter in einer anderen als einer aufrechten Position gelagert oder eingesetzt wird.

Erfindungsgemäß ist es möglich, von außen visuell den oberen Spiegel und dadurch den Füllstand von flüssigem Narkosemittel im Narkosemitteltank zu ermitteln. Ein Benutzer oder auch eine Kamera in Verbindung mit einer Bildauswerteeinheit können den Füllstand von außen ermitteln. Um diese Ermittlung zu ermöglichen, umfasst der

Narkosemittelbehälter zusätzlich eine visuelle Inspektionseinheit. Diese visuelle Inspektionseinheit ist durchsichtig und ermöglicht ein Blick von außen auf das Innere des Narkosemitteltanks. Die visuelle Inspektionseinheit kann ein Schauglas in der Wandung des Narkosemitteltanks umfassen. Möglich ist auch, dass die visuelle Inspektionseinheit ein Schaurohr aus einem durchsichtigen Material umfasst. Dieses Schaurohr steht dergestalt in mindestens einer Fluidverbindung mit dem Narkosemitteltank, dass der obere Spiegel von flüssigem Narkosemittel im Schaurohr auf der gleichen horizontalen Ebene ist wie der obere Spiegel von flüssigem Narkosemittel im Narkosemitteltank.

Erfindungsgemäß ist die visuelle Inspektionseinheit aus einem transparenten Werkstoff, der einen Anteil von mindestens 70 Gew-% Quarz (Siliziumdioxid, SiO₂) aufweist. Der Quarz-Anteil beträgt bevorzugt mindestens 80 Gew-%, besonders bevorzugt mindestens 90 Gew-%, insbesondere mindestens 99 Gew-%.

Falls der Quarz-Anteil ausreichend groß ist, so wird der Werkstoff der visuellen Inspektionseinheit in vielen Fällen nicht in relevanter Weise von flüssigem Narkosemittel angegriffen. In internen Versuchen haben die Erfinder festgestellt, dass bei einem Quarz-Anteil von mindestens 80 Gew-% oft weder eine nennenswerte Einwirkung des flüssigen Narkosemittels auf die visuelle Inspektionseinheit noch eine Einwirkung des Werkstoffs der visuellen Inspektionseinheit auf das flüssige Narkosemittel auftritt.

In einer Ausgestaltung ist auf die innere Oberfläche der visuellen Inspektionseinheit eine transparente Beschichtung aufgebracht. Diese Beschichtung befindet sich zwischen der visuellen Inspektionseinheit einerseits und dem Innenraum und daher dem flüssigen Narkosemittel im Narkosemitteltank andererseits. In vielen Fällen ist eine solche Beschichtung insbesondere dann erforderlich oder wenigstens sinnvoll, wenn der Quarz-Anteil unter 80 Gew-% liegt. Diese transparente Beschichtung ist bevorzugt aus einem Kunststoff hergestellt. Besonders bevorzugt umfasst dieser Kunststoff mindestens eines der Materialien Parylen, ein Polymer, bevorzugt ein epoxyphenolisches Polymer, Polytetrafluorethylen (PTFE), Polyolefin.

Bevorzugt sind sowohl die Wandung des Narkosemitteltanks als auch die optionale Wandung der Nachfülleinheit aus jeweils mindestens einem festen Werkstoff hergestellt. Möglich ist, dass die beiden Wandungen aus demselben Werkstoff hergestellt sind. Möglich ist auch, dass unterschiedliche Werkstoffe verwendet sind. Möglich ist auch, dass die Wandung des Narkosemitteltanks aus mindestens zwei verschiedenen Werkstoffen hergestellt ist, insbesondere dann, wenn diese Wandung zwei Schichten umfasst.

In einer Ausgestaltung umfasst der Werkstoff, aus dem die Wandung des Narkosemitteltanks hergestellt ist, mindestens eine metallische Legierung. Gemäß einer Realisierungsform dieser Ausgestaltung beträgt der Anteil von Aluminium in dieser metallischen Legierung mindestens 80 Gew-%, bevorzugt mindestens 90 Gew-%, besonders bevorzugt mindestens 95 Gew-%. Auch der Werkstoff für die optionale Wandung der Nachfülleinheit umfasst bevorzugt mindestens eine solche metallische Legierung.

Eine Wandung mit einem hohen Anteil an Aluminium ist relativ beständig gegen Korrosion und in vielen Fällen auch relativ beständig gegen ein flüssiges Narkosemittel. Außerdem weist Aluminium ein geringeres spezifisches Gewicht auf als viele andere Metalle. Darüber hinaus lässt sich ein Rohteil aus einem Werkstoff mit einem hohen Aluminium-Anteil häufig leichter in eine gewünschte Form bringen als ein Rohteil aus einem anderen Werkstoff, insbesondere durch ein Verfahren des Druckgießens oder des Strangpressens.

In einer anderen Ausgestaltung umfasst der Werkstoff, aus dem die Wandung des Narkosemitteltanks hergestellt ist, mindestens einen Kunststoff. Kunststoff kann in der Regel nicht korrodieren und weist oft ein relativ geringes Gewicht auf, und in vielen Fällen lässt sich ein Verfahren des Gießens mithilfe einer Urform anwenden, um die Wandung herzustellen. Häufig lässt sich eine Wandung mit einer komplizierten Geometrie leichter mithilfe eines Gießverfahrens herstellen als mit einem anderen Verfahren. Bevorzugt umfasst der Kunststoff mindestens eines der folgenden Materialien: ein Polyamid, ein Polyphenylsulfid, ein Polyether-Ether-Keton (PEEK).

Möglich ist eine Kombination dieser beiden Ausgestaltungen. Die Wandung umfasst zwei Schichten. Die eine Schicht umfasst eine metallische Legierung, die andere Schicht einen Kunststoff. Die erfindungsgemäße Beschichtung mit Nickel und Phosphor ist auf die innere Oberfläche der inneren Wandung aufgetragen. Bevorzugt befindet sich die Schicht mit der metallischen Legierung zwischen der erfindungsgemäßen Beschichtung und der Schicht mit dem Kunststoff. Diese Anordnung reduziert weiter die Gefahr von Korrosion.

Die Erfindung betrifft weiterhin eine Verwendung des erfindungsgemäßen Narkosemittelbehälters als Bestandteil eines Narkosemitteldosierers sowie einen Narkosemitteldosierer mit einem erfindungsgemäßen Narkosemittelbehälter. Der Narkosemitteldosierer umfasst den erfindungsgemäßen Narkosemittelbehälter und weiterhin einen Narkosemittelverdampfer mit einer Einspeise-Vorrichtung. Die Einspeise-Vorrichtung steht wenigstens zeitweise in einer Fluidverbindung mit dem erfindungsgemäßen Narkosemittelbehälter, sodass flüssiges Narkosemittel vom Narkosemittelbehälter zur Einspeise-Vorrichtung fließen kann. Möglich ist, dass diese Fluidverbindung zeitweise unterbrochen ist, beispielsweise durch ein angesteuertes Ventil oder ein sonstiges Stellglied. Die Einspeise-Vorrichtung vermag empfangenes flüssiges Narkosemittel in eine Kammer des Narkosemittelverdampfers einzuspeisen, beispielsweise einzuspritzen oder einzufüllen. Der Narkosemittelverdampfer vermag in dieser Kammer aus dem flüssigen Narkosemittel gasförmiges Narkosemittel zu erzeugen, beispielsweise durch Erhitzen und / oder Verdunsten.

Die Erfindung betrifft weiterhin einen Gasgemisch-Erzeuger
- mit einem Narkosemitteldosierer, der so wie gerade beschrieben aufgebaut ist und daher einen erfindungsgemäßen Narkosemittelbehälter umfasst, und
- einem Gasmischer.

Der Gasmischer steht wenigstens zeitweise in einer Fluidverbindung mit dem Narkosemitteldosierer, so dass gasförmiges Narkosemittel vom Narkosemitteldosierer zum Gasmischer fließen kann. Außerdem steht der Gasmischer wenigstens zeitweise in einer Fluidverbindung mit einer Quelle für ein Trägergas, wobei dieses Trägergas Sauerstoff ist oder umfasst. Der Gasmischer vermag aus dem empfangenen Trägergas und dem empfangenen gasförmigen Narkosemittel ein Gasgemisch zu erzeugen, welches Sauerstoff und flüssiges Narkosemittel umfasst. Bevorzugt vermischt der Gasmischer das gasförmige Narkosemittel mit dem Trägergas, so dass das Gasgemisch idealerweise über seine räumliche Ausdehnung homogen ist, also überall den gleichen Anteil an Narkosemittel aufweist.

Die Erfindung betrifft weiterhin ein System zur künstlichen Beatmung eines Patienten. Der Patient ist wenigstens zeitweise mit einer patientenseitigen Koppeleinheit verbunden oder lässt sich mit einer solchen verbinden. Bevorzugt umfasst die patientenseitige Koppeleinheit eine Atemmaske oder einen Tubus oder einen Katheter. Zwischen dem Beatmungssystem und der patientenseitigen Koppeleinheit ist wenigstens zeitweise eine Fluidverbindung hergestellt oder lässt sich herstellen.

Das Beatmungssystem umfasst einen erfindungsgemäßen Gasgemisch-Erzeuger und damit einen erfindungsgemäßen Narkosemittelbehälter sowie eine Fluidförderereinheit, beispielsweise eine Pumpe oder eine Kolben-ZylinderEinheit oder ein Gebläse oder auch einen manuell zu betätigenden Beatmungsbeutel. Der Gasgemisch-Erzeuger vermag ein Gasgemisch umfassend Sauerstoff und mindestens ein gasförmiges Narkosemittel zu erzeugen. Die Fluidförderereinheit vermag das Gasgemisch durch die Fluidverbindung hindurch zu der patientenseitigen Koppeleinheit zu fördern. Bevorzugt ist ein Beatmungskreislauf hergestellt, d.h. ausgeatmete Atemluft kann durch die patientenseitige Koppeleinheit hindurch zurück zum Beatmungssystem fließen. Der Beatmungskreislauf reduziert die Gefahr, dass ein gasförmiges Narkosemittel in die Umgebung oder in eine stationäre Infrastruktur eines Krankenhauses austritt.

In einer Ausgestaltung umfasst das Beatmungssystem ein Beatmungsgerät. Dieses Beatmungsgerät führt eine Abfolge von Beatmungshüben durch und fördert in jedem Beatmungshub jeweils eine Menge des Gasgemischs mit Sauerstoff und Narkosemittel zur patientenseitigen Koppeleinheit. Der Gasgemisch-Erzeuger kann ein Bestandteil dieses Beatmungsgeräts sein.

Verschiedene Verfahren sind möglich, um einen erfindungsgemäßen Narkosemittelbehälter herzustellen. Bevorzugt wird zunächst die Wandung des Narkosemitteltanks hergestellt. In einer Ausgestaltung wird zunächst ein Bauteil hergestellt, welches die Wandung des Narkosemitteltanks und die Wandung der Nachfülleinheit umfasst, wobei die beiden Wandungen bevorzugt fest und fluiddicht miteinander verbunden sind.

**In** einer Ausgestaltung wird die Beschichtung von innen auf die Wandung aufgetragen, beispielsweise aufgesprüht. **In** einer bevorzugten Ausgestaltung wird hingegen die Wandung des Narkosemitteltanks oder das Bauteil mit den beiden Wandungen relativ zu einem Tauchbad bewegt, insbesondere in ein Tauchbad abgesenkt, bevorzugt vollständig abgesenkt. Das Tauchbad stellt eine Flüssigkeit bereit, welche Nickel und Phosphor aufweist. Nach der relativen Bewegung umgibt diese Flüssigkeit bevorzugt vollständig die Wandung oder das Bauteil. Der pH-Wert der bereitgestellten Flüssigkeit legt den Anteil von Phosphor an der herzustellenden Beschichtung fest. Daher wird vorab abhängig von einem gewünschten Bereich für den Phosphor-Anteil ein pH-Wert für diese Flüssigkeit hergeleitet und vorgegeben. Bevorzugt enthält die Flüssigkeit Natriumhydrogenphosphat (NaH₂PO₂) und ein Nickelsulfat, beispielsweise NiSO₄, sowie mindestens ein geeignetes Lösungsmittel.

Die erfindungsgemäße Beschichtung bildet sich im Tauchbad auf beiden Seiten der Wandung, wobei Ionen an der Wandung abgeschieden werden und wobei die Beschichtung langsam wächst. Bevorzugt wird eine untere Schranke für die geforderte Schichtdicke vorgegeben. Die Wandung oder das Bauteil mit den beiden Wandungen wird mindestens solange im Tauchbad belassen, bis die tatsächliche Schichtdicke der Beschichtung die geforderte untere Schranke erreicht hat.

Die Ausgestaltung mit dem Tauchbad führt in vielen Fällen dazu, dass eine gleichmäßige Schichtdicke über die gesamte Ausdehnung der Wandung oder sogar des gesamten Bauteils erzielt wird. Auch dann, wenn die Wandung eine relativ komplizierte Geometrie aufweist, überdeckt eine in einem Tauchbad erzeugte erfindungsgemäße Beschichtung häufig die gesamte innere Oberfläche der Wandung. Die Verweildauer der Wandung oder des Bauteils im Tauchbad legt die erzielte Schichtdicke fest. Außerdem erfordert die Verwendung eines Tauchbads oft eine Herstellungsvorrichtung, die nur relativ wenige bewegte Teile aufweist. Erforderlich ist im Wesentlichen lediglich, das Tauchbad bereitzustellen, das zu beschichtende Bauteil vorab zu reinigen, relativ zum Tauchbad zu bewegen, insbesondere in das Tauchbad abzusenken und später wieder aus dem Tauchbad zu entnehmen.

Möglich ist, ein galvanisches Verfahren oder ein Verfahren des Eloxierens zu verwenden, um im Tauchbad die erfindungsgemäße Beschichtung zu bilden. In einer Ausgestaltung wird die Beschichtung im Tauchbad gemäß Chemisch Nickel verwendet. Ein Verfahren gemäß Chemisch Nickel erfordert nicht, dass an ein zu beschichtendes Bauteil ein Strom angelegt wird. Daher kann eine zu beschichtende Wandung auch aus einem Werkstoff hergestellt sein, der nicht oder nur wenig elektrisch leitend ist, beispielsweise aus einem starren Kunststoff. Weiterhin werden eine Energiequelle und elektrische Energie eingespart.

Im Folgenden wird die Erfindung anhand eines Ausführungsbeispiels beschrieben. Hierbei zeigt
- Figur 1: schematisch ein System zur künstlichen Beatmung eines Patienten;
- Figur 2: schematisch einen einzelnen Narkosemitteldosierer;
- Figur 3: schematisch in einer Querschnittsdarstellung einen Narkosemitteltank;
- Figur 4: schematisch in einer Ansicht von vorn den Narkosemitteltank von Figur 3 mit einem Schaurohr.

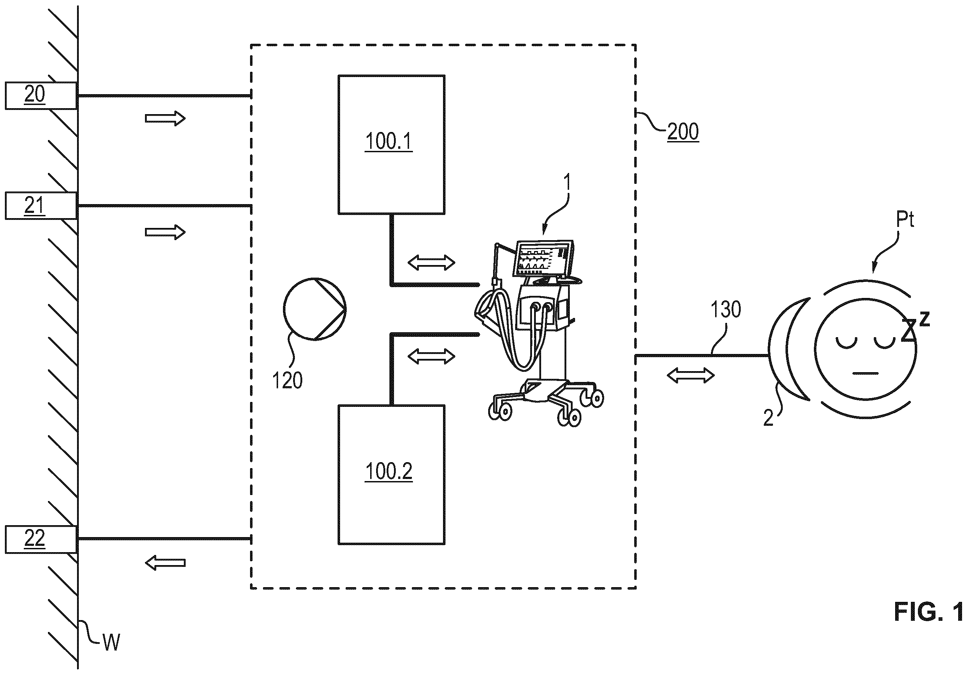

Figur 1 zeigt schematisch ein System 200 zur künstlichen Beatmung eines Patienten Pt. Der Patient Pt ist mit einer patientenseitigen Koppeleinheit 2 verbunden, beispielsweise mit einer Atemmaske auf dem Gesicht oder einem Tubus oder einem Katheter im Körper des Patienten Pt.

Das Beatmungssystem 200 führt eine Abfolge von Beatmungshüben durch. In jedem Beatmungshub wird ein Beatmungs-Gasgemisch umfassend Sauerstoff und mindestens ein Narkosemittel durch eine Fluidverbindung 130 zu der patientenseitigen Koppeleinheit 2 gefördert. Wegen des Narkosemittels im zugeführten Gasgemisch ist der Patient Pt sediert oder sogar narkotisiert. Die vom Patienten Pt ausgeatmete Luft wird wieder zurück zum Beatmungssystem 200 geführt, damit kein Narkosemittel in die Umgebung austritt. Dadurch wird ein Beatmungskreislauf zwischen dem Beatmungssystem 200 und der patientenseitigen Koppeleinheit 2 hergestellt. Eine Fluidfördereinheit in Form einer Pumpe 120 hält einen Gasfluss in diesem Beatmungskreislauf aufrecht

Das Beatmungssystem 200 umfasst ein Anästhesiegerät 1, welches die Beatmungshübe ausführt, und zwei Gasgemisch-Erzeuger 100.1 und 100.2. Die beiden Gasgemisch-Erzeuger 100.1, 100.2 sind lösbar mit dem Anästhesiegerät 1 verbunden. Die Pumpe 120 gehört zum Anästhesiegerät 1. Jeder Gasgemisch-Erzeuger 100.1, 100.2 wird vom Anästhesiegerät 1 mit einem Trägergas umfassend Sauerstoff versorgt und erzeugt ein Narkotisierungs-Gasgemisch umfassend das Trägergas und mindestens ein Narkosemittel. Das Anästhesiegerät 1 erzeugt unter Verwendung des Narkotisierungs-Gasgemischs das Beatmungs-Gasgemisch und fördert das Beatmungs-Gasgemisch zur patientenseitigen Koppeleinheit 2. Der Anteil von Sauerstoff in dem Beatmungs-Gasgemisch kann der gleiche sein wie der Anteil von Sauerstoff in Atemluft. Möglich ist, dass das Narkotisierungs-Gasgemisch als Beatmungs-Gasgemisch verwendet wird. Optional erhöht das Anästhesiegerät 1 den Anteil von Sauerstoff in dem Beatmungs-Gasgemisch.

In der Regel wird ein Gasgemisch-Erzeuger 100.1 oder 100.2 verwendet, um einen Patienten Pt zu sedieren oder zu narkotisieren. Der andere Gasgemisch-Erzeuger 100.2 oder 100.1 ist ebenfalls mit dem Anästhesiegerät 1 verbunden und wird zwar aktuell nicht verwendet, steht aber zur Verfügung, um bei Bedarf sofort verwendet zu werden. In der Regel ist es möglich, rasch von dem einen Gasgemisch-Erzeuger 100.1 zu dem anderen Gasgemisch-Erzeuger 100.2 umzuschalten, ohne die Narkotisierung des Patienten Pt zu unterbrechen.

Figur 1 zeigt weiterhin einen Versorgungsanschluss 20 für das Trägergas und einen Versorgungsanschluss 21 für Druckluft, wobei beide Versorgungsanschlüsse 20, 21 in einer Wand W angeordnet sind und zu einem stationären Versorgungssystem gehören. Im Beatmungskreislauf wird überschüssiges Gas erzeugt, welches zu einem Entsorgungsanschluss 22 in der Wand W geleitet und dort aufgenommen wird.

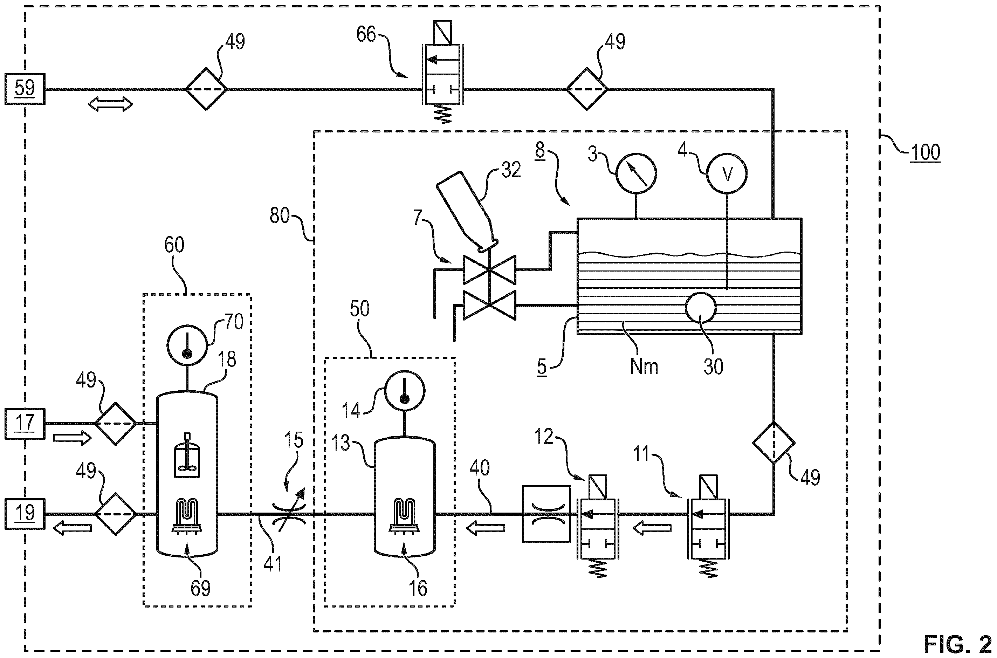

Figur 2 zeigt schematisch einen einzelnen Gasgemisch-Erzeuger 100. Dieser umfasst einen Narkosemitteldosierer 80 und einen Gasmischer 60. Über einen Einlass 17 wird das Trägergas dem Gasmischer 60 zugeführt. Der Narkosemitteldosierer 80 erzeugt gasförmiges Narkosemittel, das ebenfalls dem Gasmischer 60 zugeführt wird. Der Gasmischer 60 erzeugt aus dem gasförmigen Narkosemittel und dem Trägergas das Narkotisierungs-Gasgemisch. Das erzeugte Narkotisierungs-Gasgemisch wird über einen Auslass 19 abgeleitet und zur patientenseitigen Koppeleinheit 2 gefördert, vgl. Figur 1.

Flüssiges Narkosemittel Nm wird in einem Narkosemitteltank 5 eines Narkosemittelbehälters 8 des Narkosemitteldosierers 80 vorrätig gehalten. Möglich ist, dass der Narkosemitteldosierer 80 einen weiteren Narkosemitteltank (nicht gezeigt) umfasst, wobei dieser weitere Narkosemitteltank in einer Fluidverbindung mit dem Narkosemitteltank 5 steht und daher nicht notwendigerweise eine eigene verschließbare Nachfülleinheit umfasst. Diese Fluidverbindung fungiert als die Nachfülleinheit des weiteren Narkosemitteltanks.

In eine Wand des Narkosemitteltanks 5 ist ein Schauglas 30 eingelassen. Ein Benutzer oder auch eine Kamera können von außen durch das Schauglas 30 hindurch den aktuellen Füllstand des flüssigen Narkosemittels im Narkosemitteltank 5 visuell feststellen. In einer Ausgestaltung befindet sich außen auf dem Schauglas 30 eine Schutzschicht, die das Schauglas 30 vor mechanischen Beschädigungen von außen schützt. Ein Füllstands-Sensor 4 misst ein Maß für den aktuellen Füllstand des flüssigen Narkosemittels Nm im Narkosemitteltank 5.

Flüssiges Narkosemittel Nm lässt sich durch einen verschließbaren Stutzen 7 nachfüllen. Der Stutzen 7 fungiert als die Nachfülleinheit des Narkosemitteltanks 5 und umfasst einen Adapter, auf den sich ein Behälter zum Nachfüllen von flüssigem Narkosemittel fluiddicht aufsetzen lässt. Beispielhaft ist als Nachfüllbehälter eine Flasche 32 mit flüssigem Narkosemittel zum Nachfüllen gezeigt. Die Flasche 32 lässt sich dergestalt auf einen Adapter am Stutzen 7 aufsetzen, dass eine fluiddichte Verbindung hergestellt wird und das flüssige Narkosemittel aus der Flasche 32 durch den Stutzen 7 hindurch nach unten in den Narkosemitteltank 5 fließen kann. In der gezeigten Ausführungsform ist der Stutzen 7 an einer Seitenwand des Narkosemitteltanks 5 befestigt. Er kann auch im Deckel 10 des Narkosemitteltanks 5 angeordnet sein.

Oberhalb des Spiegels des flüssigen Narkosemittels Nm ist im Narkosemitteltank 5 ein Gasgemisch umfassend Narkosemittel vorhanden. Der Siedepunkt mancher häufig verwendeter Narkosemittel legt unterhalb von 40 °C. Insbesondere deshalb tritt im Inneren des Narkosemittelbehälters 8 ein Überdruck gegenüber dem Umgebungsdruck auf. Im Ausführungsbeispiel ist der Narkosemittelbehälter 8 so ausgestaltet, dass er einem Überdruck bis zu einer konstruktionsbedingten Überdruckschranke standhalten kann. Diese Überdruckschranke liegt zwischen 1 bar und 50 bar, bevorzugt zwischen 1 bar und 20 bar.

Ein Drucksensor 3 misst ein Maß für den Druck dieses Gasgemischs. Über eine Leitung ist der Narkosemitteltank 5 mit einem Anschluss 59 verbunden. Der Druck im Narkosemitteltank 5 lässt sich mithilfe eines Proportionalventils 66 verändern. Bevorzugt empfängt ein nicht gezeigtes signalverarbeitendes Steuergerät Messwerte vom Drucksensor 3 und steuert abhängig vom gemessenen Druck im Narkosemitteltank 5 das Proportionalventil 66 an. Das Steuergerät steuert das Proportionalventil 66 mit dem Regelungsziel an, dass der tatsächliche Druck im Narkosemitteltank 5 einem vorgegebenen zeitlichen Druckverlauf folgt.

Flüssiges Narkosemittel Nm fließt durch eine Verdampfer-Zuführleitung 40 zu einer Verdampfer-Kammer 13 eines Narkosemittelverdampfers 50. In dieser Leitung 40 sind ein ansteuerbares Proportionalventil 11 sowie eine Einspeise-Vorrichtung in Form eines ansteuerbaren Einspritzventils 12 angeordnet. Das Steuergerät steuert das Proportionalventil 11 an und steuert dadurch den Volumenfluss von flüssigem Narkosemittel Nm durch die Leitung 40. Das Einspritzventil 12 spritzt flüssiges Narkosemittel Nm in die Verdampfer-Kammer 13 des Narkosemittelverdampfers 50 ein. Eine ansteuerbare Heizung 16 trägt dazu bei, das flüssige Narkosemittel Nm in der Verdampfer-Kammer 13 zu verdampfen. Ein Temperatursensor 14 misst ein Maß für die Temperatur in der Verdampfer-Kammer 13.

Das gasförmige Narkosemittel fließt von der Verdampfer-Kammer 13 durch eine Mischer-Zuführleitung 41 mit einem pneumatischen Widerstand 15, der bevorzugt ansteuerbar ist, in eine Mischkammer 18 des Gasmischers 60. In dieser Mischkammer 18 wird das gasförmige Narkosemittel mit einem Trägergas vermischt. Eine ansteuerbare Heizung 69 vermag das Gasgemisch in der Mischkammer 18 zu heizen. Ein Temperatursensor 70 misst ein Maß für die Temperatur in der Mischkammer 70.

Außerdem sind in Figur 2 mehrere Filter 49 gezeigt.

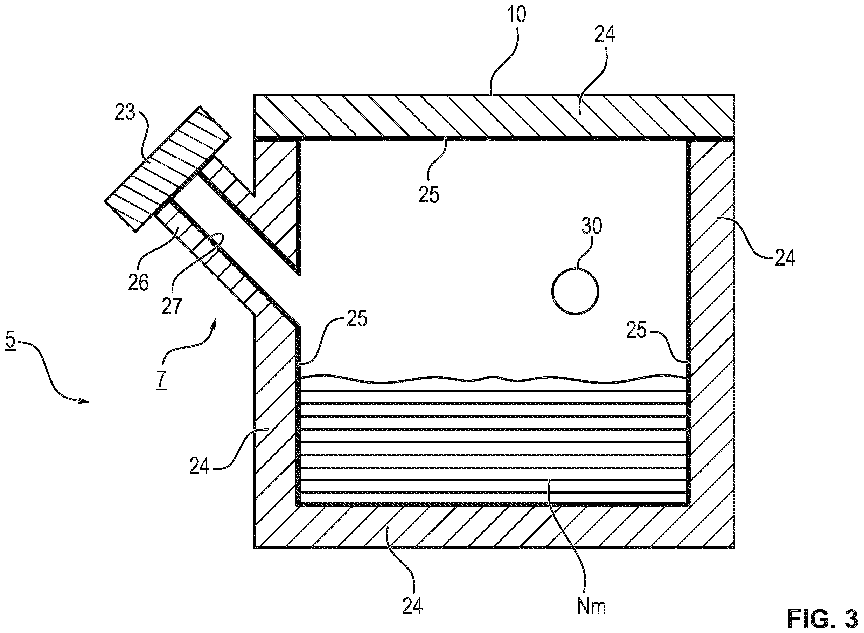

Figur 3 zeigt schematisch einen Querschnitt durch den Narkosemittelbehälter 8, wobei der Narkosemittelbehälter 8 den Narkosemitteltank 5, den Stutzen 7, den Verschluss 23 und das Schauglas 30 umfasst. Die Geometrie des Narkosemittelbehälters 8 ist vereinfacht dargestellt. Eine Wanne des Narkosemitteltanks 5 wird von einem Deckel 10 fluiddicht verschlossen. Im regulären Betrieb bleibt dieser Deckel 10 auf der Wanne des Narkosemitteltanks 5. Er kann z.B. zwecks einer Wartung geöffnet werden. Mit "dem Narkosemitteltank 5" sind im Folgenden die Wanne und der Deckel 10 gemeint. Der Stutzen 7 ist von einem Verschluss 23 fluiddicht verschlossen. Der Verschluss 23 lässt sich vom Stutzen 7 abnehmen, insbesondere um flüssiges Narkosemittel Nm in den Narkosemitteltank 5 nachzufüllen, ohne den Deckel 10 abnehmen zu müssen.

Der Narkosemitteltank 5 umfasst eine Wandung 24. In der gezeigten Realisierungsform wird diese Wandung 24 von der Wanne und dem Deckel 10 gebildet. Der Stutzen 7 umfasst eine Wandung 26. Die jeweilige Wandstärke der Wandung 24, 26 ist so festgelegt, dass der Narkosemittelbehälter 8 einem Überdruck in seinem Inneren bis zu der oben erwähnten Überdruckschranke standhalten kann. Bevorzugt liegt die Wandstärke zwischen 4 mm und 30 mm.

In einer Ausgestaltung wird die Wandung 24, 26 des Narkosemittelbehälters 8 als ein Bauteil hergestellt. In einer anderen Ausgestaltung werden zwei Hälften der Wandung 24, 26 getrennt voneinander hergestellt. Diese beiden Hälften werden anschließend durch ein Laserschweißen oder durch eine sonstige Fügetechnik miteinander verbunden. Ein Verfahren, um die Wandung 24, 26 des Narkosemittelbehälters 8 dergestalt herzustellen, wird in DE 10 2004 041 448 B3 beschrieben.

Unterschiedliche Verfahren sind möglich, wie die Wandung 24, 26 oder auch die beiden Hälften hergestellt werden. Möglich ist, die Wandung 24, 26 aus einem flüssigen Material durch ein Gießverfahren, bevorzugt durch Druckgießen, herzustellen. Möglich ist auch, die Wandung 24, 26 durch ein Verfahren des Strangpressens aus mindestens einem Blech herzustellen. Falls ein Verfahren des Strangpressen oder Gießens angewendet wird, so liegt die Wandstärke bevorzugt zwischen 4 mm und 23 mm. In einer anderen Ausgestaltung wird die Wandung 24, 26 durch Fräsen hergestellt. Die Wandstärke kann dann oberhalb von 23 mm liegen.

Mindestens zwei dieser Verfahren lassen sich auch miteinander kombinieren. Beispielsweise werden einige Teile, beispielsweise Verbindungsstücke zwischen dem Stutzen 7 und dem Narkosemitteltank 5, durch Fräsen hergestellt und die übrigen Teile durch Druckgießen oder Strangpressen. Anschließend werden die gefrästen Teile durch Laserschweißen oder eine sonstige Fügeverbindung mit den übrigen Teilen verbunden. Möglich ist, dass diejenigen Teile der Wandung 24, 26, die durch Strangpressen oder Gießen hergestellt werden, eine geringere Wandstärke aufweisen als die Teile, die durch Fräsen hergestellt werden.

Bevorzugt sind die Wandungen 24 und 26 aus demselben Werkstoff hergestellt. Möglich ist, dass ein Werkstoff für das Druckgießen oder für das Strangpressen und ein anderer Werkstoff für das Fräsen verwendet wird.

Unterschiedliche Werkstoffe für die Wandung 24, 26 sind möglich. **In** einer bevorzugten Ausgestaltung ist der Werkstoff eine metallische Legierung, die einen Anteil von mindestens 80 % Aluminium (Al) aufweist, bevorzugt von mindestens 90 %, besonders bevorzugt von 95 %. Weil der Anteil von Aluminium mindestens 80 % beträgt, weist die Wandung 24, 26 ein relativ geringes Gewicht auf und lässt sich relativ leicht in eine gewünschte Form bringen. Außerdem weist Aluminium eine ausreichend hohe Korrosionsbeständigkeit auf.

Besonders bevorzugt wird als Werkstoff für das Strangpressen oder Druckgießen eine Aluminium-Legierung gemäß EN AW-6063 verwendet. Dieser Werkstoff für die Wandung 24 hat einen Anteil zwischen 0,45 und 0,9 Gew-% Magnesium (Mg), einen Anteil zwischen 0,2 und 0,6 Gew-% Silizium (Si), einen Anteil von 0,35 Gew-% Eisen (Fe) sowie weitere Elemente mit geringeren Anteilen. Für das Fräsen wird besonders bevorzugt eine Aluminium-Legierung gemäß EN AW-5083 verwendet. Der Anteil an Magnesium (Mg) liegt zwischen 4 und 4,9 Gew-%, der Anteil an Mangan (Mn) zwischen 0,4 und 1 Gew-%, der von Silizium (Si) und von Eisen (Fe) jeweils 0,4 Gew-%.

Die metallische Legierung kann auch einen Anteil von Magnesium (Mg) von mindestens 80 Gew-%, bevorzugt von mindestens 90 Gew-% aufweisen. Bevorzugt enthält diese Legierung einen Anteil von Aluminium, der zwischen 6 Gew-% und 12 Gew-% liegt, sowie einen Anteil von Zink und Mangan, der jeweils unter 1 Gew-% liegt. Die metallische Legierung kann auch einen Anteil von Messing von mindestens 80 Gew-% aufweisen, bevorzugt von mindestens 90 Gew-%. Bekanntlich ist Messing eine Legierung mit mindestens 50 Gew-% Kupfer (Cu) und höchstens 40 Gew-% Zink (Zn).

Möglich ist auch, anstelle einer metallischen Legierung einen Kunststoff zu verwenden. Wenn die Wandung 24, 26 aus Kunststoff und nicht aus einem metallischen Legierung hergestellt ist, so ist sie nicht magnetisch und auch nicht magnetisierbar. Außerdem kann sie dann nicht korrodieren und weist in vielen Fällen ein geringeres Gewicht als eine Wandung 24, 26 aus einer metallischen Legierung auf. Eine Wandung 24, 26 aus Kunststoff wird bevorzugt durch ein Gießverfahren, besonders bevorzugt durch Druckgießen, hergestellt. Bevorzugt wird mindestens einer der folgenden Kunststoffe verwendet:
- ein Polyamid,
- ein Polyphenylsulfid (PPS) oder
- ein Polyether-Ether-Keton (PEEK, auch als PEAK bezeichnet).

Gewünscht wird, dass die Wandung 24, 26 durchgehend aus einem homogenen Werkstoff ist. In der Praxis können aber Poren in der Wandung 24, 26 auftreten. Bevorzugt wird die Wandung 24, 26 so hergestellt, dass der maximale Durchmesser einer Pore höchstens 30 µm beträgt.

In einer Ausgestaltung ist der Verschluss 23 aus einem biegsamen Kunststoff hergestellt. Dadurch füllt der Verschluss 23 einerseits die gesamte Querschnittsfläche des Stutzen 7 aus und lässt sich andererseits zusammendrücken, um den Verschluss 23 aus dem Stutzen 7 zu ziehen, oder wird zusammengedrückt, wenn der Verschluss 23 aus dem Stutzen 7 gezogen wird. Bevorzugt umfasst der Werkstoff, aus dem der Verschluss 23 hergestellt ist, ein Polyphenylensulfid (PPS), besonders bevorzugt ein mit Glasfaser verstärktes Polyphenylensulfid. Der Verschluss 23 kann auch ein starres Teil mit einem Außengewinde umfassen, wobei das Außengewinde in ein Innengewinde des Stutzen 7 eingreift. Bevorzugt umfasst der starre Verschluss 23 weiterhin eine Dichtung.

Der Narkosemitteltank 5 vermag ein flüssiges Narkosemittel aufzunehmen. Möglich ist, dass derselbe Narkosemitteltank 5 nacheinander unterschiedliche Narkosemittel aufnimmt. Beim Nachfüllen fließt flüssiges Narkosemittel Nm durch den Stutzen 7 in den Narkosemitteltank 5. Nachfolgend wird dargelegt, welche Anforderungen daraus resultieren, dass der Narkosemitteltank 5 ein flüssiges Narkosemittel aufnimmt, und wie diese Anforderungen erfindungsgemäß erfüllt werden.

Ein häufig verwendetes Narkosemittel ist unter den Bezeichnungen Sevofluran bekannt geworden. Sevofluran hat die chemische Summenformel (CF3)₂CHOCHF₂ und die chemische Bezeichnung 1,1,1,3,3,3-hexafluoro-2-Fluoromethoxy-Propan. Weitere häufig verwendete Narkosemittel haben die Bezeichnungen Isofluran und Desfluran.

Bekannt ist, dass flüssige Narkosemittel, beispielsweise die gerade angegebenen, chemisch aggressiv sind. Daher kommen als Materialien, die in Kontakt mit flüssigem Narkosemittel kommen, nur Materialien mit einer ausreichenden chemischen Beständigkeit gegen flüssige Narkosemittel in Betracht. Die Erfinder haben außerdem in internen Versuchen festgestellt, dass eine Wandung 24, 26 mit einem hohen Anteil an Aluminium sich zwar gut fertigen lässt, aber bei ungünstigen Umständen eine unerwünschte Einwirkung auf ein flüssiges Narkosemittel Nm im Narkosemitteltank 5 hat, insbesondere wenn eines der gerade genannten Narkosemittel verwendet wird. Insbesondere kann die metallische Legierung der Wandung 24 das Narkosemittel Nm im Narkosemitteltank 5 chemisch verändern oder dessen narkotisierende Wirkung herabsetzen oder sogar das Narkosemittel Nm zersetzen. Eine chemische Einwirkung der Wandungs-Legierung auf das flüssige Narkosemittel Nm kann dazu führen, dass sogenannte Lewis-Säuren gebildet werden. Die Bildung dieser Lewis-Säuren kann dazu führen, dass schädliche Substanzen, insbesondere Flusssäure (HF), gebildet wird. Die Gefahr dieser unerwünschten Einwirkung tritt insbesondere dann ein, wenn das flüssige Narkosemittel Nm relativ lange im Narkosemitteltank 5 verbleibt oder wenn der Narkosemittelbehälter 8 einer relativ hohen Umgebungstemperatur von oberhalb 35° C ausgesetzt ist.

Auf die Innenseite der Wandung 24 des Narkosemitteltanks 5 einschließlich des Deckels 10 ist eine Beschichtung 25 aufgebracht, die zwischen der Wandung 24 und dem flüssigen Narkosemittel Nm im Narkosemitteltank 5 angeordnet ist. Die Beschichtung 25 überdeckt die gesamte Innenseite der Wandung 24 und verhindert weitgehend, dass das flüssige Narkosemittel Nm in einen Kontakt mit der Wandung 24 kommt. Im Ausführungsbeispiel ist auf die Innenseite der Wandung 26 des Stutzens 7 eine Beschichtung 27 aufgebracht, die die gesamte Innenseite der Wandung 26 überdeckt. Bevorzugt bildet die Beschichtung 25, 27 eine durchgehende Beschichtung für die gesamte Innenwand des Narkosemittelbehälters 8. Zwei mögliche Ausnahmen: Die innere Oberfläche des Verschlusses 23 und die des Schauglas 30 sind frei von dieser Beschichtung 25, 27.

Möglich ist auch, dass nur die innere Oberfläche des Narkosemitteltanks 5 mit der erfindungsgemäßen Beschichtung 25 versehen ist und die innere Oberfläche des Stutzen 7 gar nicht beschichtet ist oder eine andere Beschichtung aufweist. Insbesondere dann, wenn der Stutzen 7 im oder nahe am Deckel 10 angeordnet ist, kommt der Stutzen 70 kürzer mit einem flüssigen Narkosemittel Nm in Kontakt, beispielsweise beim Nachfüllen, als die Wanne des Narkosemitteltanks 5.

Idealerweise verhindert die Beschichtung 25, 27 vollständig, dass ein flüssiges Narkosemittel im Narkosemittelbehälter 8 in Kontakt mit der Wandung 24, 26 kommt. In der Praxis bedeckt die Beschichtung 25, 27 die Wandung 24, 26 nicht vollständig und lückenfrei, sodass trotz der Beschichtung 25, 27 ein Kontakt zwischen der Wandung 24, 26 und dem flüssigen Narkosemittel Nm auftritt. Eine mögliche Ursache sind Poren in der Wandung 24, 26, deren maximaler Durchmesser größer ist als die Schichtdicke der Beschichtung 25, 27. In vielen Fällen lässt sich aber erreichen, dass die Größe dieser verbleibenden Kontaktfläche höchstens 5 %, in manchen Fällen sogar höchstens 1 %, der gesamten Fläche der Wandung 24, 26 beträgt.

Die Schichtdicke der Beschichtung 25, 27 liegt zwischen 0,5 und 80 µm, bevorzugt zwischen 10 µm und 20 µm. Besonders bevorzugt weist die Beschichtung 25, 27 eine gleichmäßige Schichtdicke von 15 µm ± 2 µm auf.

Als Werkstoff für die Beschichtung 25, 27 wird erfindungsgemäß eine Legierung aus Nickel (Ni) und Phosphor (P) und optional weiteren Komponenten verwendet. Diese Legierung ist ausreichend chemisch beständig gegen ein flüssiges Narkosemittel im Narkosemittelbehälter 8 und übt nicht eine unerwünschte Einwirkung auf das flüssige Narkosemittel Nm aus. Der Anteil an Phosphor in dieser Legierung beträgt mindestens 3 Gew-% und höchstens 15 Gew-%. Bevorzugt beträgt der Anteil von Phosphor mindestens 10 Gew-% und höchstens 13 Gew-%. Eine solche Beschichtung wird auch als Nickel Phosphor (NiP) oder auch als "Chemisch Nickel" bezeichnet. Dank des Anteils an Phosphor ist die Beschichtung 25, 27 relativ beständig gegen Verschleiß und Korrosion.

Falls der Anteil an Phosphor oberhalb von 10 Gew-% liegt, weist die Beschichtung 25 außerdem ein völlig amorphes Gefüge auf. Daher ist die Gefahr relativ gering, dass in der Beschichtung 25 Inhomogenitäten wie Korngrenzen oder ausgeschiedene Phasen auftreten. Weiterhin ist die Gefahr gering, dass beim Herstellen der Beschichtung 25, 27 Kristalle gebildet werden, welche zu einer unebenen Oberfläche der Beschichtung 25 führen können.

Nachfolgend werden verschiedene Alternativen beschrieben, um einen erfindungsgemäßen Narkosemittelbehälter 8 herzustellen. Bei allen diesen Alternativen wird zunächst ein Bauteil hergestellt, welches die beiden Wandungen 24, 26 und das optionale Sichtfenster 30 umfasst, aber noch keine erfindungsgemäße Beschichtung auf der inneren Oberfläche.

Möglich ist, die Beschichtung 25, 27 auf die Innenwand des bereitgestellten Bauteils 24, 26, 30 aufzusprühen. Denkbar ist auch, eine geeignete Flüssigkeit in das Innere des bereitgestellten Bauteils 24, 26, 30 zu füllen, diese Flüssigkeit dort zu belassen, bis sich die Beschichtung 25, 27 gebildet hat, und dann die Flüssigkeit wieder auszugießen.

**In** einer bevorzugten Ausgestaltung wird die Beschichtung 25, 27 hingegen in einem Tauchbad mit einer Flüssigkeit umfassend Nickel und Phosphor erzeugt. Das Bauteil mit der Wandung 24, 26 und dem optionalen Sichtfenster 30 wird in dieses Tauchbad abgesenkt, und zwar bevorzugt so, dass das Bauteil 24, 26, 30 vollständig in der Flüssigkeit untergetaucht ist. Durch eine chemische oder elektrochemische Reaktion bildet sich die Beschichtung 25, 27 auf der Wandung 24, 26.

Falls ein Tauchbad verwendet wird, so wird nicht nur die Innenseite der Wandung 24, 26 beschichtet, sondern auch die Außenseite. Mögliche Bohrungen, Ausbuchtungen, Hinterschneidungen sowie optionale Leitungen werden im Tauchbad ebenfalls beschichtet. Diese Beschichtung auf der Außenseite erhöht in vielen Fällen die chemische und mechanische Beständigkeit der Wandung 24, 26.

Das Beschichtungsverfahren mithilfe eines Tauchbads führt dazu, dass die Schichtdicke der Beschichtung 25, 27 über die gesamte Ausdehnung der Wandung 24, 26 hindurch eine relativ gleichbleibende Dicke aufweist. **In** vielen Fällen lässt sich erreichen, dass die Dicke der Beschichtung 25, 27 um maximal ± 5 µm oder sogar nur um ± 3 µm räumlich variiert. Oft lässt sich erreichen, dass Unebenheiten in der inneren Oberfläche der Wandung 24, 26 des Narkosemittelbehälters 8 durch die Beschichtung 25, 27 ausgeglichen und sogar wenigstens ein Teil der Poren verschlossen werden.

Bevorzugt wird die Beschichtung 25, 27 in einem Tauchbad durch eine Redox-Reaktion oder durch ein galvanisches Verfahren mit einem Elektrolyten oder durch Eloxieren auf das Bauteil 24, 26, 30 aufgebracht. Das Bauteil 24, 26, 30 wird in das Tauchbad abgesenkt. Das Tauchbad umfasst beispielsweise eine Wanne und stellt eine Flüssigkeit bereit, welche Nickel und Phosphor aufweist. Bei der Redox-Reaktion, die auch unter der Bezeichnung "Chemisch Nickel" bekannt ist, werden Nickelionen mittels einer chemischen Oxidationsreaktion an der inneren Oberfläche der Wandung 24, 26 abgeschieden.

Die Redox-Reaktion erzeugt die erforderlichen Elektronen selber. Daher ist es nicht erforderlich, eine elektrische Spannung anzulegen. In manchen Fällen ist es daher möglich, die Beschichtung 25, 27 auch dann in einem Tauchbad auf die Wandung 24, 26 durch eine chemische Reaktion aufzubringen, wenn die Wandung 24, 26 aus einem Kunststoff oder einem sonstigen Werkstoff, der nicht elektrisch leitend ist, hergestellt und daher ein galvanisches Verfahren nicht möglich ist. außerdem wird in manchen Fällen keine Versorgung mit elektrischer Energie benötigt.

In einer Ausgestaltung finden während der Beschichtung zwei chemische Teilreaktionen statt, nämlich

(1) 3 NaH₂PO₂ + 3 H₂O + NiSO₄ → 3 NAH₂PO₃ + H₂SO₄ + 2 H₂ + Ni

(2) NaH₂PO₂ + Hₙₐₛ → H₂O + NaOH + P

Je kleiner der pH-Wert des Elektrolyten im Tauchbad ist, desto langsamer läuft die Teilreaktion (1) und desto schneller die Teilreaktion (2) ab. Durch eine geeignete Wahl des pH-Werts der Flüssigkeit im Tauchbads lässt sich der Anteil von Phosphor in der Beschichtung 25, 27 festlegen. Daher wird abhängig von einem gewünschten Anteil von Phosphor in der herzustellen Beschichtung 25, 27 ein pH-Wert der im Tauchbad bereitgestellten Flüssigkeit hergeleitet und vorgegeben. Die Verweildauer des Bauteils mit den beiden Wandungen 24, 26 im Tauchbad legt die erzielte Schichtdicke der Beschichtung 25, 27 fest.

Besonders bevorzugt wird die Beschichtung 25, 27 gemäß DIN EN ISO 4527 hergestellt. Die Legierung aus Nickel und Phosphor mit einem Phosphor-Anteil von bevorzugt mindestens 10 Gew-% führt zu einer übersättigten Lösung von Phosphor im Nickel.

Im Folgenden werden beispielhaft einige Verfahrensschritte dargelegt, die zu dem Herstellungsverfahren gehören, durch den der Narkosemitteltank 8 hergestellt wird.
- Zunächst wird ein Bauteil hergestellt, welches die Wandung 24, 26 und das optionale Sichtfenster 30 umfasst.
- Von diesem Bauteil werden Fette und optional Schweißgrate entfernt.
- Mindestens einmal wird eine Oxidschicht von den beiden Oberflächen der Wandung 24, 26 entfernt.
- Außerdem wird das Bauteil 24, 26, 30 mindestens einmal gespült.
- Die beiden Oberflächen der Wandung 24, 26 werden vorbehandelt, damit die erfindungsgemäße Beschichtung 25, 27 besser haftet.
- Das Bauteil mit der Wandung 24, 26 wird in ein Tauchbad verbracht, welches in flüssiger Form ein Gemisch mit Nickel und Phosphor enthält und einen vorgegebenen pH-Wert aufweist. Im Tauchbad bildet sich auf beiden Oberflächen der der Wandung 24, 26 eine wachsende Beschichtung 25, 27. Das Bauteil 24, 26, 30 wird so lange im Tauchbad belassen, bis die Schichtdicke der Beschichtung 25, 27 eine vorgegebene untere Schranke für die Schichtdicke erreicht hat. Beispielsweise wird vorab empirisch festgestellt, mit welcher Geschwindigkeit die Schichtdicke der Beschichtung 25, 27 im Tauchbad wächst, und aus der empirisch ermittelten Wachstumsgeschwindigkeit und der vorgegebenen unteren Schranke wird eine Verweildauer des Bauteils im Tauchbad hergeleitet.

Auch mindestens einige der Schritte, Fette sowie eine Oxidschicht zu entfernen, das Bauteil zu spülen und die Oberflächen vorzubehandeln, können in jeweils einem Tauchbad durchgeführt werden

Ein Benutzer kann von außen visuell den Füllstand des flüssigen Narkosemittels Nm im Narkosemitteltank 5 feststellen. Daher ist in der Realisierungsform gemäß Figur 3 in die Wandung 24 des Narkosemitteltanks 5 ein durchsichtiges Schauglas 30 eingelassen. Bevorzugt umgibt ein O-Ring das Schauglas 30 und dichtet den Spalt zwischen dem Schauglas 30 und der Wandung 24 ab. Dieser O-Ring ist aus einem elastischen Material hergestellt, welches ausreichend chemisch resistent gegen Narkosemittel ist. Bevorzugt wird als Werkstoff für den O-Ring ein Elastomer verwendet, besonders bevorzugt der Werkstoff Ethylen-Propylen-Dien-Kautschuk (EPDM).

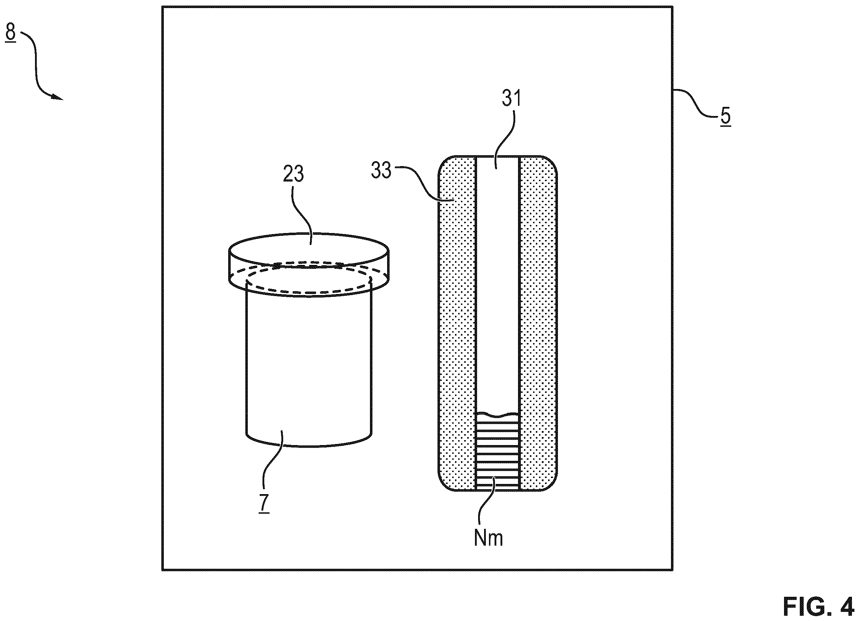

Figur 4 zeigt in einer Frontansicht eine alternative Ausführungsform. In der Darstellung gemäß Figur 4 zeigt der Stutzen 7 zum Betrachter hin. In die Wandung 24 des Narkosemitteltank 5 ist ein vertikales Schaurohr 31 fluiddicht eingelassen. Im gezeigten Beispiel bildet die Wandung 24 eine Einbuchtung 33 aus, und das Schaurohr 31 ist dergestalt von der Einbuchtung 33 umgeben, dass das Schaurohr 31 nicht außen über die Wandung 24 übersteht. Das Schaurohr 31 steht sowohl oben als auch unten in jeweils einer Fluidverbindung mit dem Inneren des Narkosemitteltanks 5, sodass der Füllstand des flüssigen Narkosemittels Nm im Narkosemitteltank 5 nach dem Prinzip der kommunizierenden Röhren mit dem Füllstand im Schaurohr 31 übereinstimmt. Beispielsweise dringt beim Nachfüllen flüssiges Narkosemittel von unten in das Schaurohr 31 ein, und Gas kann nach oben aus dem Schaurohr 31 entweichen.

Im Folgenden wird der Oberbegriff "visuelle Inspektionseinheit" für das Schauglas 30 von Figur 3 und das Schaurohr 31 von Figur 4 verwendet.

In einer bevorzugten Ausgestaltung ist die visuelle Inspektionseinheit 30, 31 aus einem Werkstoff hergestellt, der einen Anteil von mindestens 85 Gew-% Quarz (Siliziumdioxid, SiO₂) aufweist, bevorzugt einen Anteil von mindestens 95 Gew-% Quarz, besonders bevorzugt einen Anteil von mindestens 99 Gew-% Quarz. Der Werkstoff enthält außerdem Metalle, bevorzugt insbesondere Aluminium (Al). Ein Werkstoff mit einem Anteil von mindestens 85 Gew-% Quarz ist in vielen Fällen ausreichend chemisch resistent gegen Narkosemittel, sodass eine Beschichtung auf der Innenseite des Schauglases 30 zwar möglich, aber häufig nicht erforderlich ist.

In einer alternativen Ausgestaltung ist die visuelle Inspektionseinheit 30, 31 aus einem Werkstoff hergestellt, der nicht notwendigerweise einen Anteil von mindestens 85 Gew-% Quarz aufweist. Beispielsweise wird als Werkstoff ein Borosilikatglas verwendet. Borosilikatglas umfasst 70 Gew-% bis 80 Gew-% Siliziumdioxid (SiO₂), 7 Gew-% bis 13 Gew-% Bortrioxid (B₂O₃), 4 Gew-% bis 8 Gew-% Alkalioxide, beispielsweise Natriumoxid (Na₂O) oder Kaliumoxid (K₂O), sowie optional weitere Bestandteile.

Auf die innere Oberfläche der visuellen Inspektionseinheit 30, 31 ist insbesondere bei einem Quarzanteil unterhalb von 85 Gew-% eine transparente Beschichtung aus einem Kunststoff aufgebracht. Diese transparente Beschichtung auf der inneren Oberfläche verhindert eine unerwünschte Wechselwirkung zwischen der visuellen Inspektionseinheit 30, 31 und dem flüssigen Narkosemittel Nm im Narkosemitteltank 5. Bevorzugt umfasst der durchsichtige Kunststoff mindestens einen der folgenden Substanzen:
- ein Parylen,
- ein Polymer, bevorzugt ein epoxyphenolisches Polymer,
- ein durchsichtiges Polytetrafluorethylen (PTFE),
- ein Polyolefin.

**Bezugszeichenliste**

| | |
|---|---|
| 1 | Anästhesiegerät, gehört zum Beatmungssystem 200, umfasst die Fluidförderereinheit 120 |
| 2 | patientenseitige Koppeleinheit, mit dem Patienten Pt verbunden |
| 3 | Drucksensor, misst ein Maß für den Druck im Narkosemitteltank 5 |
| 4 | Füllstands-Sensor, misst ein Maß für den Füllstand des flüssigen Narkosemittels Nm im Narkosemitteltank 5 |
| 5 | Narkosemitteltank, enthält das flüssige Narkosemittel Nm, umfasst die Wandung 24 und die Beschichtung 25 |
| 7 | verschließbarer Stutzen zum Nachfüllen von flüssigem Narkosemittel Nm in den Narkosemitteltank 5 |
| 8 | Narkosemittelbehälter, umfasst den Narkosemitteltank 5 und den Stutzen 7 |
| 10 | Deckel auf der Wanne des Narkosemitteltanks 5, gehört zur Wandung 24 |
| 11 | Proportionalventil in der Leitung 40 |
| 12 | ansteuerbares Einspritzventil für flüssiges Narkosemittel, fungiert als Einspeise-Vorrichtung |
| 13 | Verdampfer-Kammer, in der flüssiges Narkosemittel Nm verdampft oder verdunstet wird, gehört zum Narkosemittelverdampfer 50 |
| 14 | Temperatursensor, misst ein Maß für die Temperatur in der Verdampfer-Kammer 13, 13, gehört zum Narkosemittelverdampfer 50 |
| 15 | pneumatischer Widerstand in der Leitung 41, ist optional ansteuerbar |
| 16 | Narkosemittel-Heizung des Narkosemittelverdampfers 50, erhitzt die Verdampfer-Kammer 13, gehört zum Narkosemittelverdampfer 50 |
| 17 | Einlass, über den das Trägergas dem Gasgemisch-Erzeuger 100 zugeführt wird |
| 18 | Mischkammer, in der das gasförmige Narkosemittel dem Trägergas zugesetzt wird, gehört zum Gasmischer 60 |
| 19 | Auslass, über den das Narkotisierungs-Gasgemisch umfassend Trägergas und Narkosemittel vom Gasgemisch-Erzeuger 100 abgeführt wird |
| 20 | Versorgungsanschluss in der Wand W für Trägergas |
| 21 | Versorgungsanschluss in der Wand W für Druckluft |
| 22 | Entsorgungsanschluss in der Wand W zur Aufnahme von überschüssigem Gasgemisch aus dem Beatmungssystem 200 |
| 23 | abnehmbarer Verschluss für den Stutzen 7 |
| 24 | Wandung des Narkosemitteltanks 5, auf der inneren Oberfläche mit der Beschichtung 25 versehen |
| 25 | Beschichtung auf der Innenwand der Wandung 24 des Narkosemitteltanks 5, aus Nickel mit mindestens 3 Gew-% und maximal 15 Gew-% Phosphor |
| 26 | Wandung des Stutzens 7, innen mit der Beschichtung 27 versehen |
| 27 | Beschichtung auf der Innenwand des Stutzens 7 |
| 30 | Schauglas zum visuellen Ermitteln des Füllstand im Narkosemitteltank 5, fluiddicht in die Wandung 24 eingelassen, aus Quarzglas oder Borosilikatglas hergestellt, umfasst optional eine innere Beschichtung |
| 31 | Schaurohr zum visuellen Ermitteln des Füllstands im Narkosemitteltank 5, in der Einbuchtung 33 angeordnet, fluiddicht mit der Wandung 24 verbunden |
| 32 | Nachfüllbehälter für flüssiges Narkosemittel Nm, lässt sich auf den Stutzen 7 aufsetzen |
| 33 | Einbuchtung in der Wandung 24, nimmt das Schaurohr 31 auf |
| 40 | Verdampfer-Zuführleitung, führt vom Narkosemitteltank 5 zur Verdampfer-Kammer 13 |
| 41 | Mischer-Zuführleitung, führt von der Verdampfer-Kammer 13 zum Mischtank 18 |
| 49 | Filter |
| 50 | Narkosemittelverdampfer, umfasst die Verdampfer-Kammer 13, die Narkosemittel-Heizung 16 und den Temperatursensor 14, erhitzen, gehört zum Narkosemitteldosierer 50 |
| 59 | Anschluss des Narkosemitteldosierers 100, über den der Druck im Narkosemitteltank 5 geregelt wird |
| 60 | Gasmischer, umfasst die Mischkammer 18, die Mischkammer-Heizung 69 und den Temperatursensor 70, erzeugt ein Narkotisierungs-Gasgemisch aus einem Trägergas und gasförmigem Narkosemittel, gehört zum Gasgemisch-Erzeuger 100 |
| 66 | Proportionalventil, mit welchem der Druck im Narkosemitteltank 5 gesteuert wird |
| 69 | Mischkammer-Heizung, vermag das Gasgemisch in der Mischkammer 18 zu erhitzen, gehört zum Gasmischer 60 |
| 70 | Temperatursensor, misst ein Maß für die Temperatur in der Mischkammer 18, gehört zum Gasmischer 60 |
| 80 | Narkosemitteldosierer, umfasst den Narkosemittelbehälter 8, den Narkosemittelverdampfer 50 und die Verdampfer-Zuführleitung 40, gehört zum Gasgemisch-Erzeuger 100 |
| 100, 100.1, 100.2 | Gasgemisch-Erzeuger, umfasst den Narkosemitteldosierer 80 und den Gasmischer 60 |
| 120 | Fluidförderereinheit in Form einer Pumpe, hält einen Strom von Gas im Beatmungskreislauf aufrecht |
| 130 | Fluidverbindung zwischen dem Beatmungssystem 200 und der patientenseitigen Koppeleinheit 2 |
| 200 | System zur künstlichen Beatmung des Patienten Pt, umfasst das Anästhesiegerät 1, die Pumpe 120 und die Narkosemitteldosierer 100.1, 100.2 |
| Nm | flüssiges Narkosemittel im Narkosemitteltank 5 |
| Pt | Patient, wird künstlich beatmet, mit der patientenseitigen Koppeleinheit 2 verbunden |
| W | Wand, trägt die Versorgungsanschlüsse 20 und 21 und den Anschluss 59 |

## Patentansprüche

1. Narkosemitteldosierer (80) umfassend
- einen Narkosemittelbehälter (8),
- eine Einspeise-Vorrichtung (12), die wenigstens zeitweise in einer Fluidverbindung (40) mit dem Narkosemittelbehälter (8) steht, und
- einen Narkosemittelverdampfer (50),
wobei der Narkosemittelbehälter (8)
- einen Narkosemitteltank (5) zur Aufnahme eines flüssigen Narkosemittels (Nm) und
- eine Nachfülleinheit (7) zum Nachfüllen von flüssigem Narkosemittel (Nm) in den Narkosemitteltank (5), insbesondere einen Stutzen (7),
umfasst,
wobei der Narkosemitteltank (5)
- eine Wandung (24) und
- eine Beschichtung (25) auf der inneren, also zum flüssigen Narkosemittel (Nm) hin zeigenden Oberfläche der Wandung (24)
umfasst,
wobei die Einspeise-Vorrichtung (12) dazu ausgestaltet ist, flüssiges Narkosemittel (Nm) aus dem Narkosemittelbehälter (8) in den Narkosemittelverdampfer (50) einzuspeisen, und
wobei der Narkosemittelverdampfer (50) dazu ausgestaltet ist, unter Verwendung des eingespeisten flüssigen Narkosemittels (Nm) gasförmiges Narkosemittel zu erzeugen,
**dadurch gekennzeichnet, dass**
die Beschichtung (25) des Narkosemitteltanks (5) aus einer Legierung besteht, die
- einen Anteil von Nickel (Ni), der zwischen 80 Gew-% und 97 Gew-% liegt, und
- einen Anteil von Phosphor (P), der zwischen 3 Gew-% und 15 Gew-% liegt,
enthält und
der Narkosemitteltank (5) eine visuelle Inspektionseinheit (30, 31) umfasst,
wobei der Füllstand von flüssigem Narkosemittel (Nm) im Narkosemitteltank (5) von außen durch die visuelle Inspektionseinheit (30, 31) hindurch sichtbar ist,
wobei die visuelle Inspektionseinheit (30, 31) aus einem transparenten Werkstoff ist und
wobei der transparente Werkstoff einen Anteil von mindestens 70 Gew-% Quarz (SiO₂) aufweist.

2. Narkosemitteldosierer (80) nach Anspruch 1,
**dadurch gekennzeichnet, dass**
der Anteil von Phosphor (P) in der Legierung, aus der die Beschichtung (25) ist,
zwischen 10 Gew-% und 13 Gew-% liegt.

3. Narkosemitteldosierer (80) nach Anspruch 1 oder Anspruch 2,
**dadurch gekennzeichnet, dass**
auf die innere Oberfläche der visuellen Inspektionseinheit (30, 31) eine transparente Beschichtung aus einem Kunststoff aufgebracht ist.

4. Narkosemitteldosierer (80) nach Anspruch 3,
**dadurch gekennzeichnet, dass**
der Kunststoff der transparenten Beschichtung auf der inneren Oberfläche
- ein Parylen,
- ein Polymer, bevorzugt ein epoxyphenolisches Polymer,
- ein durchsichtiges Polytetrafluorethylen (PTFE) und / oder
- ein Polyolefin
ist oder umfasst.

5. Narkosemitteldosierer (80) nach einem der Ansprüche 1 bis 4,
**dadurch gekennzeichnet, dass**
der oder mindestens ein Werkstoff der Wandung (24) des Narkosemitteltanks (5) mindestens eine metallische Legierung mit einem Anteil von Aluminium (Al) umfasst,
wobei der Anteil von Aluminium (Al) an der oder mindestens einer metallischen Legierung der Wandung (24) mindestens 80 Gew-% beträgt.

6. Narkosemitteldosierer (80) nach einem der Ansprüche 1 bis 5,
**dadurch gekennzeichnet, dass**
der oder mindestens ein Werkstoff der Wandung (24) des Narkosemitteltanks (5) mindestens einen Kunststoff umfasst,
wobei der oder mindestens ein Kunststoff
- ein Polyamid,
- ein Polyphenylsulfid oder
- ein Polyether-Ether-Keton
ist oder umfasst.

7. Narkosemitteldosierer (80) nach einem der Ansprüche 1 bis 6,
**dadurch gekennzeichnet, dass**
die Nachfülleinheit (7)
- eine Wandung (26) und
- eine Beschichtung (27) auf der inneren Oberfläche der Wandung (26)
umfasst,
wobei die Wandung (26) der Nachfülleinheit (7) fluiddicht mit der Wandung (24) des Narkosemitteltanks (5) verbunden ist und
wobei der Werkstoff der Beschichtung (27) der Wandung (26) der Nachfülleinheit (7)
der gleiche Werkstoff wie
der Werkstoff der Beschichtung (25) auf der inneren Oberfläche der Wandung (24) des Narkosemitteltanks (5) ist.

8. Gasgemisch-Erzeuger (100) umfassend
- einen Narkosemitteldosierer (80) nach einem der Ansprüche 1 bis 7 und
- einem Gasmischer (60), der wenigstens zeitweise in einer Fluidverbindung (41) mit dem Narkosemitteldosierer (80) steht,
wobei der Gasmischer (60) dazu ausgestaltet ist,
- ein Gasgemisch umfassend Sauerstoff und mindestens ein gasförmiges Narkosemittel zu erzeugen und
- für die Erzeugung des Gasgemischs gasförmiges Narkosemittel zu verwenden, welches vom Narkosemitteldosierer (80) erzeugt ist.

9. Beatmungssystem (200) zur künstlichen Beatmung eines Patienten (Pt),
wobei der Patient (Pt) mit einer patientenseitigen Koppeleinheit (2) verbunden oder wenigstens zeitweise verbindbar ist,
wobei das Beatmungssystem (200)
- eine Fluidförderereinheit (120) und
- einen Gasgemisch-Erzeuger (100) nach Anspruch 8
umfasst,
wobei eine Fluidverbindung (130) zwischen dem Beatmungssystem (200) und der patientenseitigen Koppeleinheit (2) hergestellt oder wenigstens zeitweise herstellbar ist,
wobei der Gasgemisch-Erzeuger (100) dazu ausgestaltet ist, ein Gasgemisch umfassend Sauerstoff und mindestens ein gasförmiges Narkosemittel zu erzeugen, und
wobei die Fluidführungseinheit (120) dazu ausgestaltet ist, das Gasgemisch durch die Fluidverbindung (130) hindurch zur patientenseitigen Koppeleinheit (2) zu fördern.

10. Herstellungs-Verfahren zum Herstellen eines Narkosemitteldosierers (80) nach einem der Ansprüche 1 bis 7,
wobei
- eine untere Schranke für die Schichtdicke der Beschichtung (25) des Narkosemitteltanks (5) vorgegeben wird und
- abhängig von einem vorgegebenen Bereich für den Anteil an Phosphor (P) in der Beschichtung (25) ein pH-Wert vorgegeben wird und
wobei das Verfahren die Schritte umfasst, dass
- die Wandung (24) des Narkosemitteltanks (25) hergestellt wird,
- die Wandung (24) relativ zu einem Tauchbad bewegt wird, insbesondere in das Tauchbad eingetaucht wird, besonders bevorzugt vollständig eingetaucht wird,
wobei das Tauchbad eine Flüssigkeit enthaltend Nickel (Ni) und Phosphor (P) bereitstellt,
wobei die Flüssigkeit den vorgegebenen pH-Wert aufweist und
wobei diese Flüssigkeit nach der Relativbewegung die Wandung (24) umgibt, bevorzugt vollständig umgibt,
- die Wandung (24) im Tauchbad belassen wird, sodass sich auf beiden Seiten der Wandung (24) jeweils eine Beschichtung bildet, und
- die Wandung (24) so lange im Tauchbad belassen wird, bis wenigstens die Beschichtung (25) auf der inneren Oberfläche der Wandung (24) eine Wandstärke erreicht, die größer als oder gleich der vorgegebenen unteren Schranke für die Schichtdicke ist.

11. Herstellungs-Verfahren nach Anspruch 10,
**dadurch gekennzeichnet, dass**
beim Schritt, die Wandung (24) im Tauchbad zu belassen, sodass sich die beiden Beschichtungen bilden,
ein Verfahren des Chemisch Nickel verwendet wird.

12. Herstellungs-Verfahren nach Anspruch 10 oder Anspruch 11,
**dadurch gekennzeichnet, dass**
ein Bauteil umfassend die Wandung (24) des Narkosemitteltanks (5) und eine Wandung (26) der Nachfülleinheit (7) dergestalt hergestellt wird, dass die beiden Wandungen (24, 26) fluiddicht miteinander verbunden sind,
das Bauteil (24, 26) relativ zum Tauchbad bewegt wird und
das Bauteil (24, 26) so lange in dem Tauchbad belassen wird, bis wenigstens die Beschichtung (25) auf der inneren Oberfläche der Wandung (24) eine Wandstärke erreicht hat, die größer oder gleich der vorgegebenen unteren Schranke für die Schichtdicke ist.

## Claims

1. Anesthetic dispenser (80) comprising
- an anesthetic container (8),
- a feed device (12) which is at least temporarily in a fluid connection (40) with the anesthetic container (8), and
- an anesthetic vaporizer (50),
the anesthetic container (8) comprising
- an anesthetic tank (5) for receiving a liquid anesthetic (Nm) and
- a refill unit (7) for refilling liquid anesthetic (Nm) into the anesthetic tank (5), in particular a nozzle (7),
the anesthetic tank (5) comprising
- a wall (24) and
- a coating (25) on the inner surface of the wall (24), i.e. the surface pointing toward the liquid anesthetic (Nm),
the feed device (12) being designed to feed liquid anesthetic (Nm) from the anesthetic container (8) into the anesthetic vaporizer (50), and
the anesthetic vaporizer (50) being designed to generate gaseous anesthetic using the fed-in liquid anesthetic (Nm),
**characterized in that**
the coating (25) of the anesthetic tank (5) consists of an alloy which contains
- a proportion of nickel (Ni) of between 80 wt.% and 97 wt.%, and
- a proportion of phosphorus (P) of between 3 wt.% and 15 wt.%,
and
the anesthetic tank (5) comprises a visual inspection unit (30, 31),
the fill level of liquid anesthetic (Nm) in the anesthetic tank (5) being visible from the outside through the visual inspection unit (30, 31),
the visual inspection unit (30, 31) being made of a transparent material and
the transparent material having a proportion of at least 70 wt.% quartz (SiO₂).

2. Anesthetic dispenser (80) according to claim 1,
**characterized in that**
the proportion of phosphorus (P) in the alloy from which the coating (25) is made is
between 10 wt% and 13 wt%.

3. Anesthetic dispenser (80) according to either claim 1 or claim 2,
**characterized in that**
a transparent coating made of a plastics material is applied to the inner surface of the visual inspection unit (30, 31).

4. Anesthetic dispenser (80) according to claim 3,
**characterized in that**
the plastics material of the transparent coating on the inner surface is or comprises
- a parylene,
- a polymer, preferably an epoxy phenolic polymer,
- a transparent polytetrafluoroethylene (PTFE) and/or
- a polyolefin.

5. Anesthetic dispenser (80) according to any of claims 1 to 4,
**characterized in that**
the or at least one material of the wall (24) of the anesthetic tank (5) comprises at least one metal alloy having a proportion of aluminum (Al),
the proportion of aluminum (Al) in the or at least one metal alloy of the wall (24) being at least 80 wt.%.

6. Anesthetic dispenser (80) according to any of claims 1 to 5,
**characterized in that**
the or at least one material of the wall (24) of the anesthetic tank (5) comprises at least one plastics material,
the or at least one plastic being or comprising
- a polyamide,
- a polyphenyl sulfide or
- a polyether ether ketone.

7. Anesthetic dispenser (80) according to any of claims 1 to 6,
**characterized in that**
the refill unit (7) comprises
- a wall (26) and
- a coating (27) on the inner surface of the wall (26),
the wall (26) of the refill unit (7) being connected in a fluid-tight manner to the wall (24) of the anesthetic tank (5) and
the material of the coating (27) of the wall (26) of the refill unit (7)
being the same material as
the material of the coating (25) on the inner surface of the wall (24) of the anesthetic tank (5).

8. Gas mixture generator (100) comprising
- an anesthetic dispenser (80) according to any of claims 1 to 7 and
- a gas mixer (60) which is at least temporarily in a fluid connection (41) with the anesthetic dispenser (80),
the gas mixer (60) being designed
- to generate a gas mixture comprising oxygen and at least one gaseous anesthetic and
- to use gaseous anesthetic generated by the anesthetic dispenser (80) to generate the gas mixture.

9. Ventilation system (200) for artificial ventilation of a patient (Pt),
wherein the patient (Pt) is connected or at least temporarily connectable to a patient-side coupling unit (2),
wherein the ventilation system (200) comprises
- a fluid conveying unit (120) and
- a gas mixture generator (100) according to claim 8,
wherein a fluid connection (130) is established or is at least temporarily establishable between the ventilation system (200) and the patient-side coupling unit (2),
wherein the gas mixture generator (100) is designed to generate a gas mixture comprising oxygen and at least one gaseous anesthetic, and
wherein the ventilation device (120) is designed to convey a gas mixture through the fluid connection (130) to the patient-side coupling unit (2).

10. Manufacturing method for manufacturing an anesthetic dispenser (80) according to any of claims 1 to 7,
wherein
- a lower limit for the layer thickness of the coating (25) of the anesthetic tank (5) is specified and
- a pH is specified depending on a specified range for the proportion of phosphorus (P) in the coating (25) and
wherein the method comprises the steps of
- manufacturing the wall (24) of the anesthetic tank (25),
- moving the wall (24) relative to a dipping bath, in particular immersing, particularly preferably completely immersing, the wall in the dipping bath,
providing the dipping bath with a liquid containing nickel (Ni) and phosphorus (P),
wherein the liquid has the specified pH and
wherein this liquid encloses, preferably completely encloses, the wall (24) after the relative movement,
- leaving the wall (24) in the dipping bath so that a coating is formed on both sides of the wall (24), and
- leaving the wall (24) in the dipping bath until at least the coating (25) on the inner surface of the wall (24) reaches a wall thickness which is greater than or equal to the specified lower limit for the layer thickness.

11. Manufacturing method according to claim 10,
**characterized in that**
in the step of leaving the wall (24) in the dipping bath so that the two coatings form,
an electroless nickel plating method is used.

12. Manufacturing method according to either claim 10 or claim 11,
**characterized in that**
a component comprising the wall (24) of the anesthetic tank (5) and a wall (26) of the refill unit (7) is manufactured in such a way that the two walls (24, 26) are interconnected in a fluid-tight manner,
the component (24, 26) is moved relative to the dipping bath and
the component (24, 26) is left in the dipping bath until at least the coating (25) on the inner surface of the wall (24) has reached a wall thickness which is greater than or equal to the specified lower limit for the layer thickness.

## Revendications

1. Doseur d'anesthésique (80) comprenant
- un récipient pour anesthésique (8),
- un dispositif d'injection (12) qui est au moins temporairement en liaison fluidique (40) avec le récipient pour anesthésique (8), et
- un vaporisateur pour anesthésique (50),
dans lequel le récipient pour anesthésique (8) comprend
- un réservoir d'anesthésique (5) destiné à contenir un anesthésique (Nm) liquide et
- une unité de recharge (7) pour la recharge de l'anesthésique (Nm) liquide dans le réservoir d'anesthésique (5), en particulier une buse (7),
dans lequel le réservoir d'anesthésique (5) comprend
- une paroi (24) et
- un revêtement (25) sur la surface intérieure, c'est-à-dire orientée vers l'anesthésique (Nm) liquide, de la paroi (24)
dans lequel le dispositif d'injection (12) est conçu pour injecter l'anesthésique (Nm) liquide dans le vaporisateur pour anesthésique (50) à partir du réservoir d'anesthésique (8), et
dans lequel le vaporisateur pour anesthésique (50) est conçu pour générer un anesthésique gazeux à l'aide de l'anesthésique (Nm) liquide injecté,
**caractérisé en ce que**
le revêtement (25) du réservoir d'anesthésique (5) est constitué d'un alliage qui contient
- une proportion de nickel (Ni) comprise entre 80 % et 97 % en poids, et
- une proportion de phosphore (P) comprise entre 3 % et 15 % en poids,
et
le réservoir d'anesthésique (5) comprend une unité d'inspection (30, 31) visuelle,
dans lequel le niveau de remplissage d'anesthésique (Nm) liquide dans le réservoir d'anesthésique (5) est visible de l'extérieur à travers l'unité d'inspection (30, 31) visuelle,
dans lequel l'unité d'inspection (30, 31) visuelle est faite en un matériau transparent, et
dans lequel le matériau transparent présente une proportion d'au moins 70 % en poids de quartz (SiO₂).

2. Doseur d'anesthésique (80) selon la revendication 1,
**caractérisé en ce que**
la proportion de phosphore (P) dans l'alliage dont est constitué le revêtement (25),
se situe entre 10 % et 13 % en poids.

3. Doseur d'anesthésique (80) selon la revendication 1 ou la revendication 2,
**caractérisé en ce que**
un revêtement transparent en un plastique est appliqué sur la surface intérieure de l'unité d'inspection (30, 31) visuelle.

4. Doseur d'anesthésique (80) selon la revendication 3,
**caractérisé en ce que**
le plastique du revêtement transparent sur la surface intérieure est ou comprend
- un parylène,
- un polymère, de préférence un polymère époxy-phénolique,
- un polytétrafluoroéthylène (PTFE) transparent et/ou
- une polyoléfine.

5. Doseur d'anesthésique (80) selon l'une des revendications 1 à 4,
**caractérisé en ce que**
le matériau ou au moins un matériau de la paroi (24) du réservoir d'anesthésique (5) comprend au moins un alliage métallique comportant une proportion d'aluminium (Al),
dans lequel la proportion d'aluminium (Al) dans l'alliage métallique ou au moins un alliage métallique de la paroi (24) est d'au moins 80 % en poids.

6. Doseur d'anesthésique (80) selon l'une des revendications 1 à 5,
**caractérisé en ce que**
le matériau ou au moins un matériau de la paroi (24) du réservoir d'anesthésique (5) comprend au moins un plastique,
dans lequel le plastique ou au moins un plastique est ou comprend
- un polyamide,
- un polyphénylsulfure, ou
- une polyéther-éther-cétone.

7. Doseur d'anesthésique (80) selon l'une des revendications 1 à 6,
**caractérisé en ce que**
l'unité de recharge (7) comprend
- une paroi (26) et
- un revêtement (27) sur la surface intérieure de la paroi (26),
dans lequel la paroi (26) de l'unité de recharge (7) est reliée de manière étanche aux fluides à la paroi (24) du réservoir d'anesthésique (5) et
dans lequel le matériau du revêtement (27) de la paroi (26) de l'unité de recharge (7) est
le même matériau que
le matériau du revêtement (25) sur la surface intérieure de la paroi (24) du réservoir d'anesthésique (5).

8. Générateur de mélange gazeux (100) comprenant
- un doseur d'anesthésique (80) selon l'une des revendications 1 à 7 et
- un mélangeur de gaz (60) qui est au moins temporairement en liaison fluidique (41) avec le doseur d'anesthésique (80),
dans lequel le mélangeur de gaz (60) est conçu pour
- générer un mélange gazeux comprenant de l'oxygène et au moins un anesthésique gazeux, et
- utiliser, pour la génération du mélange gazeux, un anesthésique gazeux généré par le doseur d'anesthésique (80).

9. Système d'assistance respiratoire (200) pour la respiration artificielle d'un patient (Pt),
dans lequel le patient (Pt) est relié ou peut être relié au moins temporairement à une unité d'accouplement (2) côté patient,
dans lequel le système d'assistance respiratoire (200) comprend
- une unité de transport de fluide (120) et
- un générateur de mélange gazeux (100) selon la revendication 8,
dans lequel une liaison fluidique (130) est établie ou peut être établie au moins temporairement entre le système d'assistance respiratoire (200) et l'unité d'accouplement (2) côté patient,
dans lequel le générateur de mélange gazeux (100) est conçu pour générer un mélange gazeux comprenant de l'oxygène et au moins un anesthésique gazeux, et
dans lequel l'unité de transport de fluide (120) est conçue pour transporter le mélange gazeux à travers la liaison fluidique (130) jusqu'à l'unité d'accouplement (2) côté patient.

10. Procédé de fabrication pour la fabrication d'un doseur d'anesthésique (80) selon l'une des revendications 1 à 7,
dans lequel
- une limite inférieure pour l'épaisseur de couche du revêtement (25) du réservoir d'anesthésique (5) est prédéfinie et
- en fonction d'une plage prédéfinie pour la proportion de phosphore (P) dans le revêtement (25), une valeur de pH est prédéfinie et
dans lequel le procédé comprend les étapes selon lesquelles
- la paroi (24) du réservoir d'anesthésique (25) est fabriquée,
- la paroi (24) est déplacée par rapport à un bain d'immersion, est en particulier immergée dans le bain d'immersion, est de manière particulièrement préférée complètement immergée,
dans lequel le bain d'immersion fournit un liquide contenant du nickel (Ni) et du phosphore (P),
dans lequel le liquide présente la valeur de pH prédéfinie et
dans lequel ce liquide entoure, de préférence complètement, la paroi (24) après le déplacement relatif,
- la paroi (24) est laissée dans le bain d'immersion, de manière à ce qu'un revêtement se forme respectivement de part et d'autre de la paroi (24), et
- la paroi (24) est laissée dans le bain d'immersion jusqu'à ce qu'au moins le revêtement (25) sur la surface intérieure de la paroi (24) atteigne une épaisseur de paroi qui est supérieure ou égale à la limite inférieure prédéfinie pour l'épaisseur de couche.

11. Procédé de fabrication selon la revendication 10,
**caractérisé en ce que**
lors de l'étape consistant à laisser la paroi (24) dans le bain d'immersion de manière à ce que les deux revêtements se forment,
un procédé de nickelage chimique est utilisé.

12. Procédé de fabrication selon la revendication 10 ou la revendication 11,
**caractérisé en ce que**
un composant comprenant la paroi (24) du réservoir d'anesthésique (5) et une paroi (26) de l'unité de recharge (7) est fabriqué de telle sorte que les deux parois (24, 26) sont reliées entre elles de manière étanche aux fluides,
le composant (24, 26) est déplacé par rapport au bain d'immersion, et
le composant (24, 26) est laissé dans le bain d'immersion jusqu'à ce qu'au moins le revêtement (25) sur la surface intérieure de la paroi (24) ait atteint une épaisseur de paroi qui est supérieure ou égale à la limite inférieure prédéfinie pour l'épaisseur de couche.
